(19) Europäisches Patentamt
European Patent Office
Office européen des brevets

(11) EP 4 772 903 A1

(12) EUROPEAN PATENT APPLICATION
published in accordance with Art. 153(4) EPC

(43) Date of publication:
08.07.2026 Bulletin 2026/28

(21) Application number: 24859381.6

(22) Date of filing: 06.08.2024

(51) International Patent Classification (IPC):
G01S 7/40 (2006.01)    A61B 5/11 (2006.01)
G01S 7/02 (2006.01)    G01S 13/34 (2006.01)

(52) Cooperative Patent Classification (CPC):
A61B 5/11; G01S 7/02; G01S 7/40; G01S 13/34;
G01S 13/88

(86) International application number:
PCT/JP2024/028133

(87) International publication number:
WO 2025/047341 (06.03.2025 Gazette 2025/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.08.2023 JP 2023140490

(71) Applicant: Kyocera Corporation
Kyoto-shi, Kyoto 612-8501 (JP)

(72) Inventors:
• KURODA Jun
Kyoto-shi, Kyoto 612-8501 (JP)
• MURAKAMI Youhei
Kyoto-shi, Kyoto 612-8501 (JP)
• KAWAJI Satoshi
Kyoto-shi, Kyoto 612-8501 (JP)

(74) Representative: Viering, Jentschura & Partner
mbB
Patent- und Rechtsanwälte
Am Brauhaus 8
01099 Dresden (DE)

(54) ELECTRONIC DEVICE, METHOD FOR CONTROLLING ELECTRONIC DEVICE, AND PROGRAM

(57) An electronic device is provided. The electronic device includes a transmission antenna unit, a transmission signal processing unit, and a transmission transfer line part. The transmission antenna unit is configured to have a plurality of transmission channels. The transmission signal processing unit is configured to generate a transmission signal to be transmitted from the transmission antenna unit. The transmission transfer line part is configured to electrically connect the transmission antenna unit and the transmission signal processing unit. The transmission signal processing unit is configured to correct a phase difference between channels of signals transmitted in the transmission transfer line part.

FIG. 11

**Description**

CROSS-REFERENCE TO RELATED APPLICATIONS

This application claims priority based on Japanese Patent Application No. 2023-140490 filed August 30, 2023, the entire disclosure of which is hereby incorporated by reference.

TECHNICAL FIELD

The present disclosure relates to an electronic device, a method of controlling an electronic device, and a program.

BACKGROUND OF INVENTION

In fields such as the automotive and related industries, for example, technologies for measuring values such as the distance between a vehicle and a given object are gaining importance. In particular, recent years have seen various research into radio detection and ranging (RADAR) technology, which measures values such as the distance to an obstacle or other object by transmitting radio waves, such as millimeter waves, and receiving reflected waves back from the object. The importance of technologies for measuring such distances and the like is expected to increase further with the development of technologies for assisting drivers with driving and technologies related to self-driving, in which driving is partially or fully automated.

Various research is also underway into technologies that can be utilized to perform sensing using various sensors. For example, Patent Literature 1 proposes an in-vehicle device capable of transferring power via radio waves to a sensor that detects biological information and the like pertaining to a user riding in a vehicle. In the future, sensors based on technologies such as radar described above may not only be mounted on moving bodies such as automobiles, but may also be mounted on various portions of various objects, depending on the application.

CITATION LIST

PATENT LITERATURE

Patent Literature 1: Japanese Unexamined Patent Application Publication No. 2022-20270

SUMMARY

In an embodiment, an electronic device is provided. The electronic device includes a transmission antenna unit, a transmission signal processing unit, and a transmission transfer line part. The transmission antenna unit is configured to have a plurality of transmission channels. The transmission signal processing unit is configured to generate a transmission signal to be transmitted from the transmission antenna unit. The transmission transfer line part is configured to electrically connect the transmission antenna unit and the transmission signal processing unit. The transmission signal processing unit is configured to correct a phase difference between channels of signals transmitted in the transmission transfer line part.

In an embodiment, a method of controlling an electronic device is provided. The electronic device includes a transmission antenna unit, a transmission signal processing unit, and a transmission transfer line part. The transmission antenna unit is configured to have a plurality of transmission channels. The transmission signal processing unit is configured to generate a transmission signal to be transmitted from the transmission antenna unit. The transmission transfer line part is configured to electrically connect the transmission antenna unit and the transmission signal processing unit. The method includes correcting, by the transmission signal processing unit, a phase difference between channels of signals transmitted in the transmission transfer line part.

In an embodiment, a program is provided. The program causes an electronic device to execute a process. The electronic device includes a transmission antenna unit, a transmission signal processing unit, and a transmission transfer line part. The transmission antenna unit is configured to have a plurality of transmission channels. The transmission signal processing unit is configured to generate a transmission signal to be transmitted from the transmission antenna unit. The transmission transfer line part is configured to electrically connect the transmission antenna unit and the transmission signal processing unit. The process includes correcting, by the transmission signal processing unit, a phase difference between channels of signals transmitted in the transmission transfer line part.

BRIEF DESCRIPTION OF THE DRAWINGS

FIG. 1 is a diagram for describing how an electronic device according to a comparative example of an embodiment is used.

FIG. 2 is a function block diagram schematically illustrating a configuration of an electronic device according to a comparative example of an embodiment.

FIG. 3 is a diagram for describing the composition of a signal processed by an electronic device according to a comparative example of an embodiment.

FIG. 4 is a diagram for describing the processing of a signal by an electronic device according to a comparative example of an embodiment.

FIG. 5 is a diagram for describing the processing of a signal by an electronic device according to a comparative example of an embodiment.

FIG. 6 is a diagram for describing the processing of a signal by an electronic device according to a comparative example of an embodiment.

FIG. 7 schematically illustrates an example of the antenna arrangement and the operating principle of an antenna array of an electronic device according to a comparative example of an embodiment.

FIG. 8 illustrates an example of the antenna arrangement in an antenna array of an electronic device according to a comparative example of an embodiment.

FIG. 9 is a diagram illustrating the exterior of an electronic device according to a comparative example of an embodiment.

FIG. 10 is a diagram illustrating an example of an internal configuration of an electronic device according to a comparative example of an embodiment.

FIG. 11 is a diagram schematically illustrating a configuration of an electronic device according to an embodiment.

FIG. 12 is a diagram illustrating an example of the exterior of an electronic device according to an embodiment.

FIG. 13 is a Smith chart illustrating an example of operations by an electronic device according to a comparative example of an embodiment.

FIG. 14 is a Smith chart illustrating an example of operations by an electronic device according to an embodiment.

FIGs. 15A and 15B are diagrams illustrating S-parameters of an electronic device according to a comparative example of an embodiment.

FIGs. 16A and 16B are diagrams illustrating S-parameters of an electronic device according to an embodiment.

FIGs. 17A and 17B are diagrams illustrating a partially enlarged view of the graphs illustrated in FIGs. 16A and 16B, respectively.

FIG. 18 is a flowchart for describing operations by an electronic device according to an embodiment.

FIG. 19 is a diagram schematically illustrating a configuration of an electronic device according to an embodiment.

FIG. 20 is a diagram for describing an electronic device according to an embodiment.

FIG. 21 is a diagram illustrating an example of use of an electronic device according to an embodiment.

FIG. 22 is a diagram illustrating an example of use of an electronic device according to an embodiment.

FIG. 23 is a diagram illustrating an example of use of an electronic device according to an embodiment.

DESCRIPTION OF EMBODIMENTS

Convenience could be enhanced by the availability of an electronic device that allows for a higher degree of freedom in implementation while also achieving good detection through the transmission and reception of radio waves, such as millimeter waves, for example. An objective of the present disclosure is to provide an electronic device, a method of controlling an electronic device, and a program that allow for enhanced convenience. According to an embodiment, an electronic device, a method of controlling an electronic device, and a program that allow for enhanced convenience can be provided. The following describes an electronic device according to an embodiment with reference to the drawings.

In the present disclosure, an "electronic device" may be a device driven by electric power. A "user" may be an entity (typically a human being) or an animal that uses an electronic device according to an embodiment and/or a system including the electronic device. The user may include an entity that monitors a human being or other subject by using an electronic device according to an embodiment. The "subject" may be an entity (subject of monitoring, such as a human being or an animal, for example) to be monitored using an electronic device according to an embodiment. The user may also include the subject.

In the present disclosure, "heartbeat" may refer to the pulsation of the heart. "Pulsation" may refer to a rhythmic contraction movement performed by the heart. The heart may be a human heart or an animal heart. The "heartbeat" and the "heart sound" may be the "heartbeat" and the "heart sound" of a human or an animal.

In the present disclosure, "heart rate" refers to the number of times the heart pulsates in a prescribed period of time. For example, the heart rate may refer to the number of pulsations per minute. Pulsations occur in the arteries when the heart pumps blood. Accordingly, the number of pulsations by the arteries may also be referred to as the "pulse rate" or simply the "pulse".

In the present disclosure, "heart sound" may refer to the sound of a beating heart. That is, the heart sound may refer to the sound that occurs when the heart contracts and dilates. The "heart sound" may consist of a low, long first sound based on ventricular muscle tension, mitral valve closure, initiation of blood ejection into the arteries, and/or acceleration of blood flow, followed by a high, short second sound derived from aortic valve closure and/or pulmonary valve closure.

In the present disclosure, the heart sound is not necessarily limited to physical sounds based on air vibrations, and may also mean the vibrations themselves caused by the beating (pulsation) of the heart. For example, in the present disclosure, the heartbeat may be assumed to imply a vibrating source, and the heart sound may be assumed to imply the vibrations themselves caused by the vibrating source. In the present disclosure, the heartbeat may also be assumed to imply the heart sound, depending on the situation.

An electronic device according to an embodiment can detect biological information such as the heartbeat of a human being or other subject present in the surroundings of the electronic device. Accordingly, anticipated situations in which an electronic device according to an embodiment is used may be, for example, specific facilities or the like used by people engaged in social activities, such as companies, hospitals, nursing homes, schools, sports gyms, and nursing care facilities. For example, it is extremely important for a company to grasp and/or manage the health status of its employees and the like. Similarly, it is extremely important for a hospital to grasp and/or manage the health status of its patients, medical personnel, and the like, and for a nursing home to grasp and/or manage the health status of its residents, staff, and the like. The situations in which an electronic device according to an embodiment is used are not limited to facilities such as companies, hospitals, and nursing homes as described above, and may be any given facility where it is desirable to grasp and/or manage the health status of a subject. The any given facility may also include a non-commercial facility, such as the home of a user. The situations in which an electronic device according to an embodiment is used are not limited to indoors and may also be outdoors. For example, the situations in which an electronic device according to an embodiment is used may also be inside means of transportation such as trains, buses, airplanes, and the like, as well as stations, boarding areas, and the like. The situations in which an electronic device according to an embodiment is used may also be a means of transportation such as an automobile, aircraft, or ship, a hotel, the home of a user, or the living room, bath, toilet, bedroom, or the like in a home. The situations in which an electronic device according to an embodiment is used may also be when measuring the heartbeats of animals such as cows, pigs, or other livestock at zoos, ranches, farms, and the like.

In a nursing facility, for example, an electronic device according to an embodiment may be used for the purpose of detecting or monitoring biological information such as the heartbeat of a subject such as a person in need of nursing or care. If an abnormality is found in the biological information such as the heartbeat of a subject such as a person in need of nursing or care, for example, an electronic device according to an embodiment may also issue a predetermined warning to the person in question and/or another person. Consequently, according to an electronic device according to an embodiment, the person in question and/or a staff member of a nursing care facility or the like may recognize that an abnormality is found in the pulse of a subject such as a person in need of nursing or care, for example. On the other hand, if an abnormality is not found (for example, the heartbeat is found to be normal) in the heartbeat of a subject such as a person in need of nursing or care, for example, an electronic device according to an embodiment may also issue a notification to that effect to the person in question and/or another person. Consequently, according to an electronic device according to an embodiment, the person in question and/or a staff member of a nursing care facility or the like may recognize that the pulse is normal for a subject such as a person in need of nursing or care, for example. An electronic device according to an embodiment may also use the heartbeat of a subject of monitoring to estimate the heartbeat interval (the RR interval (RRI) in an electrocardiogram), detection of distracted driving such as falling asleep at the wheel, and predictive signs thereof, estimation of level of alertness, estimation of level of fatigue, or identification of the subject of monitoring.

An electronic device according to an embodiment may also detect the pulse of an animal other than a human being as the subject. As an example, the electronic device according to an embodiment described hereinafter is described as detecting the pulse of a human being using a sensor based on a technology like millimeter-wave radar.

An electronic device according to an embodiment may be installed in any stationary object, and may also be installed in any moving object. An electronic device according to an embodiment can transmit a transmission wave from a transmitting antenna to the surroundings of the electronic device. An electronic device according to an embodiment can receive, from a receiving antenna, a reflected wave resulting from the transmission wave being reflected. At least one of the transmitting antenna or the receiving antenna may be provided in the electronic device, and may also be provided in a radar sensor, for example.

In the following, an electronic device according to an embodiment is described as stationary. An electronic device according to an embodiment may also be installed in a moving body such as an automobile, for example. By being installed in a moving body such as an automobile, for example, an electronic device according to an embodiment may detect biological information such as the heartbeat of an occupant on board the moving body. On the other hand, the subject (human being) whose pulse or other biological information is to be detected by an electronic device according to an embodiment may be stationary, moving, or moving their body while remaining stationary. Like an ordinary radar sensor, an electronic device according to an embodiment can measure the distance and the like between the electronic device and an object in the surroundings of the electronic device in situations in which the object may move. An electronic device according to an embodiment can measure the distance and the like between the electronic device and an object even if the electronic device and the object are both stationary.

To describe an electronic device according to an embodiment, the following first describes an electronic device according to a comparative example of an embodiment. First, an example of the detection of an object by an electronic device according to a comparative example of an embodiment will be described.

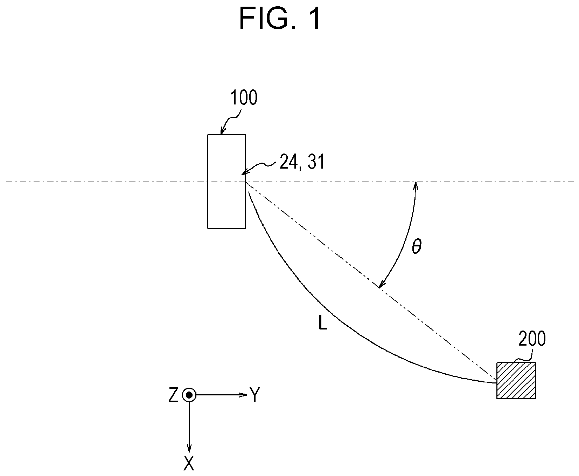

FIG. 1 is a diagram for describing an example of how an electronic device according to a comparative example of an embodiment is used. FIG. 1 illustrates an example of an electronic device provided with the functions of a sensor provided with a transmitting antenna and a receiving antenna according to an embodiment.

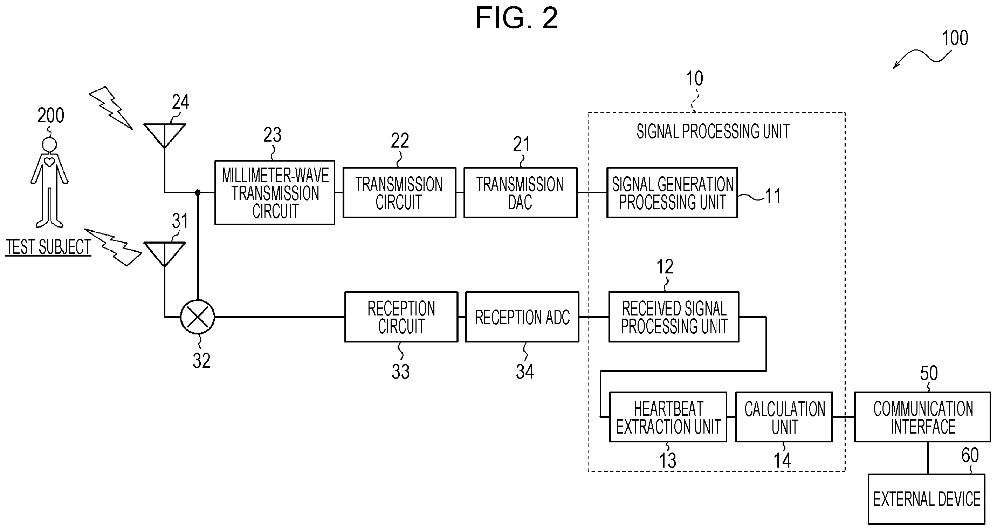

As illustrated in FIG. 1, in a comparative example of an embodiment, an electronic device 100 may be provided with a transmission unit and a reception unit. As described later, the transmission unit may be provided with a transmitting antenna array 24. The reception unit may be provided with a receiving antenna array 31. The transmitting antenna array 24 and the receiving antenna array 31 may be provided inside an enclosure of the electronic device 100, for example. Specific configurations of the electronic device 100, the transmission unit, and the reception unit will be described later. For simplicity, FIG. 1 schematically illustrates a situation in which the electronic device 100 has an enclosure inside which the transmitting antenna array 24 and the receiving antenna array 31 are provided. The electronic device 100 may also include at least one other functional unit, as appropriate, such as at least a portion of a signal processing unit 10 (FIG. 2) included in the electronic device 100. The electronic device 100 may be provided with at least one other functional unit outside the electronic device 100, such as at least a portion of a signal processing unit 10 (FIG. 2) included in the electronic device 100. In FIG. 1, the electronic device 100 may be moving, but may also be stationary without moving.

The example illustrated in FIG. 1 illustrates the electronic device 100 such that the transmission unit provided with the transmitting antenna array 24 and the reception unit provided with the receiving antenna array 31 are provided inside an enclosure of the electronic device 100. The electronic device 100 may also be provided with a plurality of transmission units and/or a plurality of reception units, for example. The transmission unit may be provided with a transmitting antenna array 24 formed from a plurality of transmitting antennas. The reception unit may be provided with a receiving antenna array 31 formed from a plurality of receiving antennas. The position where the transmission unit and/or reception unit are installed in the electronic device 100 is not limited to the position illustrated in FIG. 1, and may be another position, as appropriate. The number of transmission units and/or reception units may be any number equal to or greater than 1, according to various conditions (or requirements) such as the range and/or precision of the detection of biological information such as heartbeat by the electronic device 100.

As described later, the electronic device 100 transmits an electromagnetic wave as a transmission wave from the transmitting antenna array 24. For example, if a given object (for example, the subject 200 illustrated in FIG. 1) is present in the surroundings of the electronic device 100, at least a portion of the transmission wave transmitted from the electronic device 100 is reflected by the object to become a reflected wave. Such a reflected wave is then received by the receiving antenna array 31 of the electronic device 100, for example, whereby the electronic device 100 can detect the subject as a target.

Typically, the electronic device 100 provided with the transmitting antenna array 24 may be a radio detection and ranging (RADAR) sensor that transmits and receives radio waves. However, the electronic device 100 is not limited to a radar sensor. In a comparative example of an embodiment, the electronic device 100 may also be, for example, a sensor based on light detection and ranging (LIDAR) technology, also known as laser imaging detection and ranging, which involves light waves. Sensors such as these can be configured to include a patch antenna or the like. Since technologies such as RADAR and LIDAR are already known, a detailed description of these technologies may be simplified or omitted, as appropriate. In a comparative example of an embodiment, the electronic device 100 may also be a sensor based on a technology that detects objects by transmitting and receiving sonic or ultrasonic waves, for example.

The electronic device 100 illustrated in FIG. 1 receives from the receiving antenna array 31 a reflected wave of a transmission wave transmitted from the transmitting antenna array 24. With this arrangement, the electronic device 100 can detect a given subject 200 present within a given distance from the electronic device 100 as a target. For example, as illustrated in FIG. 1, the electronic device 100 can measure the distance L between the electronic device 100 and a given subject 200. The electronic device 100 can also measure the relative velocity between the electronic device 100 and a given subject 200. The electronic device 100 can also measure the direction (direction of arrival (hereinafter also referred to as the "angle of arrival $\theta$")) from which the reflected wave from a given subject 200 arrives at the electronic device 100.

In FIG. 1, the XY plane may be defined as the plane substantially parallel to the ground, for example. In this case, the positive direction of the Z axis illustrated in FIG. 1 may indicate the vertically upward direction. In FIG. 1, the electronic device 100 may be installed on a plane parallel to the XY plane. In FIG. 1, the subject 200 may be standing on the ground substantially parallel to the XY plane, for example.

The subject 200 may be a human being or the like present in the surroundings of the electronic device 100, for example. The subject 200 may also be a living thing other than a human being, such as an animal, present in the surroundings of the electronic device 100, for example. As described above, the subject 200 may be moving, and may also be stopped or stationary. In the present disclosure, the object to be detected by the electronic device 100 includes inanimate things such as any object, as well as living things such as people, dogs, cats, horses, and other animals. The object to be detected by the electronic device 100 according to the present disclosure may also include a target, including a person, a thing, an animal, or the like, that is detected by radar technology. In the present disclosure, a target may include a person, a thing, an animal, or the like. The following description assumes that the object such as the subject 200 present in the surroundings of the electronic device 100 is a human being (or an animal). Hereinafter, the "subject 200" is also referred to as the "test subject 200", as appropriate. In the present disclosure, the target may also be the subject 200 above.

In FIG. 1, the ratio of the size of the electronic device 100 to the size of the subject 200 does not necessarily indicate the actual ratio. In FIG. 1, the transmitting antenna array 24 of the transmission unit and the receiving antenna array 31 of the reception unit are illustrated as being installed inside an enclosure of the electronic device 100. However, in an embodiment, the transmitting antenna array 24 of the transmission unit and/or the receiving antenna array 31 of the reception unit may be installed at various positions in the electronic device 100. For example, in an embodiment, the transmitting antenna array 24 of the transmission unit and/or the receiving antenna array 31 of the reception unit may also be installed on the outside of the enclosure of the electronic device 100.

The following describes a typical example in which the transmitting antenna of the electronic device 100 transmits radio waves in a frequency band such as millimeter waves (30 GHz or higher) or quasi-millimeter waves (for example, around 20-30 GHz). On the other hand, the transmitting antenna of the electronic device 100 may also transmit radio waves with a frequency bandwidth of 4 GHz, such as from 77 GHz to 81 GHz, for example. The transmitting antenna of the electronic device 100 may also transmit radio waves of high frequency (for example, 30 GHz to 300 GHz) in or above the millimeter-wave band.

FIG. 2 is a function block diagram schematically illustrating an example configuration of the electronic device 100 according to a comparative example of an embodiment. The following describes an example of the configuration of the electronic device 100 according to a comparative example of an embodiment.

Frequency-modulated continuous-wave radar (hereinafter referred to as FMCW radar) is often used to measure distances and the like using millimeter-wave radar. In FMCW radar, a transmission signal is generated by sweeping the frequencies of the radio waves to be transmitted. Accordingly, in a millimeter-wave FMCW radar that uses radio waves in the 79 GHz frequency band, for example, the frequencies of the radio waves to be used have a frequency bandwidth of 4 GHz, such as from 77 GHz to 81 GHz, for example. Radar in the 79 GHz frequency band is characterized by having a wider usable frequency bandwidth than other millimeter/quasi-millimeter wave radars, such as radars in the 24 GHz, 60 GHz, and 76 GHz frequency bands, for example. The following describes such an embodiment as an example.

The radar scheme of the FMCW radar used in the present disclosure may include a fast-chirp modulation (FCM) scheme that transmits chirp signals on a shorter period than usual. The signal that the electronic device 100 generates is not limited to a signal of the FMCW scheme. The signal that the electronic device 100 generates may also be a signal of any of various schemes other than the FMCW scheme. A transmission signal sequence stored in any storage unit may be different depending on these various schemes. For example, in the case of a radar signal of the FMCW scheme described above, a signal of increasing frequency and a signal of decreasing frequency at each time sample may be used. Known technologies can be applied, as appropriate, for the various schemes described above, and thus a more detailed description is omitted.

As illustrated in FIG. 2, in a comparative example of an embodiment, the electronic device 100 is provided with a signal processing unit 10. The signal processing unit 10 may be provided with a signal generation processing unit 11, a received signal processing unit 12, a heartbeat extraction unit 13, and a calculation unit 14. The heartbeat extraction unit 13 may execute processing to extract a micro-Doppler component, for example. The heartbeat extraction unit 13 may also execute processing to extract an envelope of the heart sound of the test subject 200. The calculation unit 14 may execute processing to calculate a heartbeat interval (RRI) or the like of the test subject 200, for example. The calculation unit 14 may also execute processing to calculate the heartbeat or the like of the test subject 200, for example. The calculation unit 14 may further execute processing to calculate a heart rate variability (HRV) or the like of the test subject 200, for example. In this case, the calculation unit 14 may also execute processing such as performing frequency analysis of time-series data on the extracted heartbeat interval of the test subject 200. The calculation unit 14 may also execute processing to calculate the heart rate variability or the like of the test subject 200 on the basis of frequency analysis of time-series data on the heartbeat interval. The signal generation processing unit 11, received signal processing unit 12, heartbeat extraction unit 13, and calculation unit 14 will be further described later, as appropriate. In the present disclosure, the heart sound may refer to, for example, a chest vibration waveform observed directly by radar, or to chest vibrations. The heartbeat refers to the pulsations themselves of the heart. A heartbeat interval, heart rate, or the like may be calculated from the movement of the heartbeat. The heartbeat interval may refer to the time interval between a pulsation of the heart and the next pulsation of the heart.

In a comparative example of an embodiment, the electronic device 100 is provided with a transmission DAC 21, a transmission circuit 22, a millimeter-wave transmission circuit 23, and the transmitting antenna array 24 as the transmission unit. In a comparative example of an embodiment, the electronic device 100 is provided with the receiving antenna array 31, a mixer 32, a reception circuit 33, and a reception ADC 34 as the reception unit. In a comparative example of an embodiment, the electronic device 100 need not include at least one of the functional units illustrated in FIG. 2, and may also include a functional unit other than the functional units illustrated in FIG. 2. The electronic device 100 illustrated in FIG. 2 may be formed using a circuit configured in basically the same and/or similar way as a common radar using electromagnetic waves in the millimeter-wave band or the like. On the other hand, in the electronic device 100 according to a comparative example of an embodiment, the signal processing by the signal processing unit 10 may include processing different from the processing performed by a common radar of the related art.

In a comparative example of an embodiment, the signal processing unit 10 provided in the electronic device 100 can control operations by the electronic device 100 as a whole, including control of each of the functional units that make up the electronic device 100. In particular, the signal processing unit 10 performs various processing with respect to signals handled by the electronic device 100. To provide control and processing power for executing various functions, the signal processing unit 10 may include at least one processor, such as a central processing unit (CPU) or a digital signal processor (DSP). The signal processing unit 10 may be realized entirely with a single processor, with several processors, or with respectively discrete processors. The processor may be realized as a single integrated circuit. An integrated circuit is also referred to as an IC. The processor may be realized as a plurality of communicatively connected integrated circuits and discrete circuits. The processor may be realized on the basis of any of various other known technologies. In an embodiment, the signal processing unit 10 may be configured as a CPU (hardware) and a program (software) executed by the CPU, for example. The signal processing unit 10 may include a storage unit (memory) required for operations by the signal processing unit 10.

The signal generation processing unit 11 of the signal processing unit 10 generates a signal to be transmitted from the electronic device 100. In the electronic device 100 according to a comparative example of an embodiment, the signal generation processing unit 11 may generate a transmission signal such as a chirp signal (transmission chirp signal). In particular, the signal generation processing unit 11 may generate signals (linear chirp signals) whose frequency varies periodically and linearly. For example, the signal generation processing unit 11 may generate chirp signals whose frequency increases periodically and linearly from 77 GHz to 81 GHz over time. As another example, the signal generation processing unit 11 may generate a signal whose frequency periodically and linearly increases (up-chirp) from 77 GHz to 81 GHz and then decreases (down-chirp) over time. The signal that the signal generation processing unit 11 generates may also be preset in the signal processing unit 10, for example. The signal that the signal generation processing unit 11 generates may also be stored in advance in any storage unit or the like in the signal processing unit 10, for example. Since chirp signals used in technical fields such as radar are already known, a more detailed description is simplified or omitted, as appropriate. The signal generated by the signal generation processing unit 11 is supplied to the transmission DAC 21. For this reason, the signal generation processing unit 11 may be connected to the transmission DAC 21.

The transmission DAC (digital-to-analog converter) 21 functions to convert a digital signal supplied from the signal generation processing unit 11 to an analog signal. The transmission DAC 21 may include a common digital-to-analog converter. The signal converted to analog by the transmission DAC 21 is supplied to the transmission circuit 22. For this reason, the transmission DAC 21 may be connected to the transmission circuit 22.

The transmission circuit 22 functions to convert the signal converted to analog by the transmission DAC 21 to an intermediate frequency (IF) band. The transmission circuit 22 may include a common IF-band transmission circuit. The signal processed by the transmission circuit 22 is supplied to the millimeter-wave transmission circuit 23. For this reason, the transmission circuit 22 may be connected to the millimeter-wave transmission circuit 23.

The millimeter-wave transmission circuit 23 functions to transmit the signal processed by the transmission circuit 22 as a millimeter wave (RF wave). The millimeter-wave transmission circuit 23 may include a common millimeter-wave transmission circuit. For example, the millimeter-wave transmission circuit 23 may be configured to include an amplifier (amplification circuit) that amplifies the power (electrical energy) of a transmission wave (transmission signal). The signal processed by the millimeter-wave transmission circuit 23 is supplied to the transmitting antenna array 24. For this reason, the millimeter-wave transmission circuit 23 may be connected to the transmitting antenna array 24. The signal processed by the millimeter-wave transmission circuit 23 is also supplied to the mixer 32. For this reason, the millimeter-wave transmission circuit 23 may also be connected to the mixer 32.

The transmitting antenna array 24 is a plurality of transmitting antennas arranged into an array. In FIG. 2, the configuration of the transmitting antenna array 24 is illustrated in a simplified manner. The transmitting antenna array 24 transmits a signal processed by the millimeter-wave transmission circuit 23 to the outside of the electronic device 100. The transmitting antenna array 24 may include a transmitting antenna array used in a common millimeter-wave radar.

In this way, in a comparative example of an embodiment, the electronic device 100 is provided with a transmitting antenna (transmitting antenna array 24) and can transmit a transmission signal (transmission chirp signal, for example) as a transmission wave from the transmitting antenna array 24.

As an example, suppose the case in which an object such as the test subject 200 is present in the surroundings of the electronic device 100, as illustrated in FIG. 2. In this case, at least a portion of the transmission wave transmitted from the transmitting antenna array 24 is reflected by an object such as the test subject 200. The at least a portion of the transmission wave transmitted from the transmitting antenna array 24 that is reflected by an object such as the test subject 200 may be reflected toward the receiving antenna array 31.

The receiving antenna array 31 receives a reflected wave. The reflected wave may refer to at least a portion of a transmission wave transmitted from the transmitting antenna array 24 that is reflected by an object such as the test subject 200.

The receiving antenna array 31 is a plurality of receiving antennas arranged into an array. In FIG. 2, the configuration of the receiving antenna array 31 is illustrated in a simplified manner. The receiving antenna array 31 receives a reflected wave resulting from a transmission wave transmitted from the transmitting antenna array 24 being reflected. The receiving antenna array 31 may include a receiving antenna array used in a common millimeter-wave radar. The receiving antenna array 31 supplies a received signal received as a reflected wave to the mixer 32. For this reason, the receiving antenna array 31 may be connected to the mixer 32. The reflected wave (received signal) that the receiving antenna array 31 receives may be supplied to the mixer 32 after being amplified while maintaining low noise, such as by using a low-noise amplifier (LNA), for example. In FIG. 2, the LNA or the like is omitted from illustration.

The mixer 32 converts a signal (transmission signal) processed by the millimeter-wave transmission circuit 23 and a received signal received by the receiving antenna array 31 (and amplified while maintaining low noise by the LNA) to an intermediate frequency (IF) band. The mixer 32 may include a mixer used in a common millimeter-wave radar. The mixer 32 supplies a signal generated as a synthesized result to the reception circuit 33. For this reason, the mixer 32 may be connected to the reception circuit 33.

The reception circuit 33 functions to perform analog processing on a signal converted to an IF band by the mixer 32. The reception circuit 33 may include a common reception circuit that performs conversion to an IF band. The signal processed by the reception circuit 33 is supplied to the reception ADC 34. For this reason, the reception circuit 33 may be connected to the reception ADC 34.

The reception ADC (analog-to-digital converter) 34 functions to convert an analog signal supplied from the reception circuit 33 to a digital signal. The reception ADC 34 may include a common analog-to-digital converter. The signal converted to digital by the reception ADC 34 is supplied to the received signal processing unit 12 of the signal processing unit 10. For this reason, the reception ADC 34 may be connected to the signal processing unit 10.

The received signal processing unit 12 of the signal processing unit 10 functions to perform various processing on a digital signal supplied from the reception ADC 34. For example, the received signal processing unit 12 calculates the distance from the electronic device 100 to an object such as the test subject 200 on the basis of a digital signal supplied from the reception ADC 34 (distance measurement). The received signal processing unit 12 also calculates the relative velocity of an object such as the test subject 200 relative to the electronic device 100 on the basis of a digital signal supplied from the reception ADC 34 (velocity measurement). The received signal processing unit 12 further calculates the bearing angle of an object such as the test subject 200 as seen from the electronic device 100 on the basis of a digital signal supplied from the reception ADC 34 (angle measurement). Specifically, I/Q converted data may be inputted into the received signal processing unit 12. By accepting the input of such data, the received signal processing unit 12 performs a fast Fourier transform (2D-FFT) in each of the distance (range) and velocity directions. The received signal processing unit 12 may then perform false alarm suppression and fixed probability conversion by removing noise points through processing such as constant false alarm rate (CFAR). The received signal processing unit 12 can perform angle-of-arrival estimation for points that satisfy the CFAR criterion to obtain the position of an object such as the test subject 200. The information generated as a result of the distance measurement, velocity measurement, and angle measurement by the received signal processing unit 12 may be supplied to the heartbeat extraction unit 13.

The heartbeat extraction unit 13 extracts information related to heartbeat from information generated by the received signal processing unit 12. The extraction unit 13 may execute processing to extract a micro-Doppler component and extract from the information generated by the received signal processing unit 12 and heart sound envelope. Any of various approaches may be used for the operation of extracting information related to heartbeat by the heartbeat extraction unit 13. The information related to heartbeat extracted by the heartbeat extraction unit 13 may be supplied to the calculation unit 14.

The calculation unit 14 performs various arithmetic processing, computational processing, and/or the like on information related to heartbeat supplied from the heartbeat extraction unit 13. For example, the calculation unit 14 may execute processing to calculate the HRV by extracting a heartbeat interval on the basis of the concession related to heartbeat supplied from the heartbeat extraction unit 13 and analyzing the frequency of the heartbeat interval (RRI) time-series data. Any of various approaches may be using for the various arithmetic processing, computational processing, and/or the like by the calculation unit 14. Various information resulting from the arithmetic processing, computational processing, and/or the like by the calculation unit 14 may be supplied to, for example, a communication interface 50. For this reason, the calculation unit 14 and/or signal processing unit 10 may be connected to the communication interface 50.

Various information resulting from the arithmetic processing, computational processing, and/or the like by the calculation unit 14 may also be supplied to another functional unit other than the communication interface 50.

The communication interface 50 is configured to include an interface that outputs information supplied from the signal processing unit 10 to, for example, an external device 60. The communication interface 50 may output information on at least one of the position, velocity, and angle of an object such as the test subject 200 as a controller area network (CAN) or other type of signal, for example, to the external device 60 or the like. For example, information on at least one of the position, velocity, and angle of an object such as the test subject 200 may be supplied to the external device 60 or the like via the communication interface 50. For this reason, the communication interface 50 may be connected to the external device 60 or the like.

As illustrated in FIG. 2, in a comparative example of an embodiment, the electronic device 100 may be connected to the external device 60 in a wired or wireless manner via the communication interface 50. In an embodiment, the external device 60 may be configured to include a computer of any kind, a control device of any kind, and/or the like. In a comparative example of an embodiment, the electronic device 100 may be configured to include the external device 60. The external device 60 may be configured in a variety of ways according to the manner in which information on heartbeat and/or heart sound detected by the electronic device 100 is to be used. Accordingly, a more detailed description of the external device 60 is omitted.

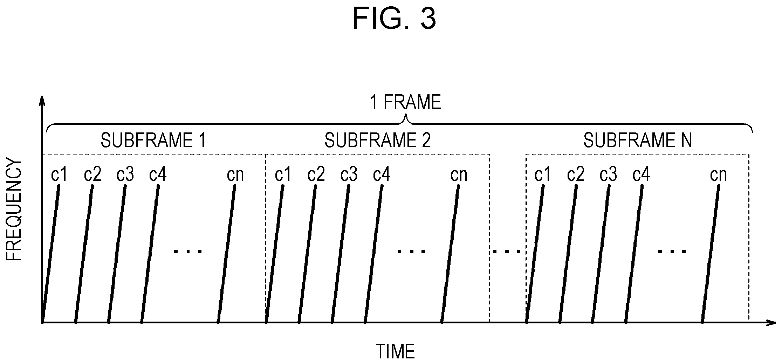

FIG. 3 is a diagram for describing an example of chirp signals generated by the signal generation processing unit 11 of the signal processing unit 10.

FIG. 3 illustrates the temporal structure of one frame in the case of using the fast-chirp modulation (FCM) scheme. FIG. 3 illustrates an example of a received signal of the FCM scheme. FCM is a scheme in which chirp signals illustrated as c1, c2, c3, c4, ..., cn in FIG. 3 are repeated at relatively short intervals (for example, equal to or greater than the round-trip time of electromagnetic waves between the radar and the target as calculated from the maximum ranging distance). In FCM, the transmission and reception processing is often divided into subframe units as illustrated in FIG. 3 for convenient signal processing of a received signal.

In FIG. 3, the horizontal axis represents elapsed time, and the vertical axis represents frequency. In the example illustrated in FIG. 3, the signal generation processing unit 11 generates linear chirp signals whose frequency varies periodically and linearly. In FIG. 3, the chirp signals are indicated as c1, c2, c3, c4, ..., cn. As illustrated in FIG. 3, in each of the chirp signals, the frequency increases linearly over time.

In the example illustrated in FIG. 3, several chirp signals such as c1, c2, c3, c4, ..., cn are included as a single subframe. That is, subframe 1, subframe 2, and so on illustrated in FIG. 3 are each configured to include several chirp signals such as c1, c2, c3, c4, ..., cn. In the example illustrated in FIG. 3, several subframes such as subframe 1, subframe 2, ..., subframe N are included as a single frame (1 frame). That is, the 1 frame illustrated in FIG. 3 is configured to include N subframes. The 1 frame illustrated in FIG. 3 may be frame 1, followed by frame 2, frame 3, and so on. These frames are each configured to include N subframes, in the same and/or similar manner as frame 1. A frame interval of given length may also be included between frames. The single frame illustrated in FIG. 3 may be around 30-50 milliseconds long, for example.

In the electronic device 100 according to a comparative example of an embodiment, the signal generation processing unit 11 may generate a transmission signal as any number of frames. In FIG. 3, some of the chirp signals are omitted from illustration. The relationship between the time and frequency of a transmission signal generated by the signal generation processing unit 11 in this way may be stored as various setting parameters in a storage unit or the like of the signal processing unit 10.

In this way, in a comparative example of an embodiment, the electronic device 100 may transmit a transmission signal formed from subframes including a plurality of chirp signals. In a comparative example of an embodiment, the electronic device 100 may transmit a transmission signal formed from frames including a given number of subframes.

The following describes the electronic device 100 as transmitting a transmission signal with a frame structure as illustrated in FIG. 3. However, the frame structure as illustrated in FIG. 3 is an example, and the chirp signals included in one subframes may be of any kind, for example. That is, in an embodiment, the signal generation processing unit 11 may generate subframes including any number of (for example, any plurality of) chirp signals. The subframe structure illustrated in FIG. 3 is also an example, and the subframes included in one frame may be of any kind, for example. That is, in an embodiment, the signal generation processing unit 11 may generate frames including any number of (for example, any plurality of) subframes. The signal generation processing unit 11 may generate signals of different frequency. The signal generation processing unit 11 may generate a plurality of discrete signals each having a different frequency f.

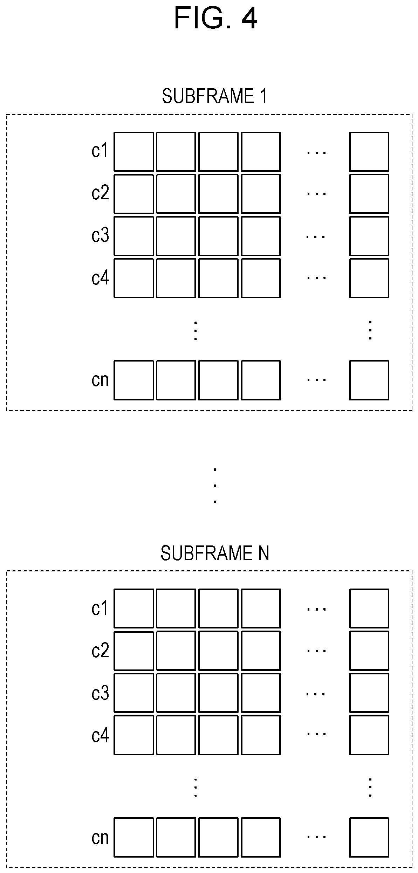

FIG. 4 illustrates another form of a portion of the subframes illustrated in FIG. 3. FIG. 4 is an illustration the result of the received signal processing unit 12 (FIG. 2) of the signal processing unit 10 performing two-dimensional fast Fourier transform (2D-FFT) processing on samples of a received signal obtained by receiving the transmission signal illustrated in FIG. 3.

As illustrated in FIG. 4, each of chirp signals c1, c2, c3, c4, ..., cn is stored in each of subframes such as subframe 1, ..., subframe N. In FIG. 4, each of the chirp signals c1, c2, c3, c4, ..., cn is formed from samples, which are indicated by the horizontally arrayed grid squares. The received signal illustrated in FIG. 4 is subjected to 2D-FFT, CFAR, integration signal processing on each subframe, and/or the like by the received signal processing unit 12 illustrated in FIG. 2.

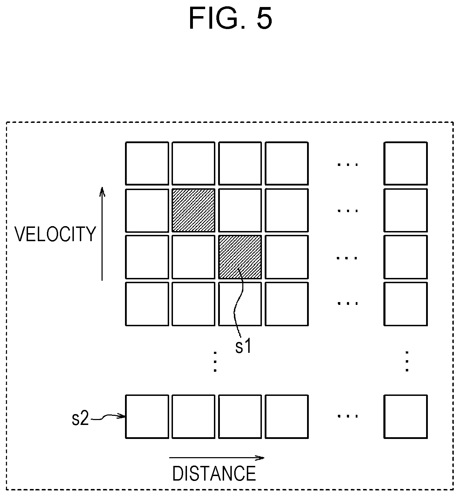

FIG. 5 illustrates an example of a point group in the range-Doppler (distance-velocity) plane calculated as a result of performing 2D-FFT, CFAR, and integration signal processing on each subframe in the received signal processing unit 12 illustrated in FIG. 2.

In FIG. 5, the horizontal direction represents range (distance), and the vertical direction represents velocity. The shaded grid square s1 illustrated in FIG. 5 illustrates a point group indicating a signal exceeding CFAR threshold processing. The non-shaded grid square s2 illustrated in FIG. 5 illustrates a bin (2D-FFT sample) with no point group, which did not exceed the CFAR threshold. Direction estimation is used to calculate the bearing, from the radar, of the calculated point group in the range-Doppler plane illustrated in FIG. 5, and the position and velocity in the two-dimensional plane are calculated as a point group indicating an object such as the test subject 200. Direction estimation may be calculated by a beamformer and/or a subspace method. Typical subspace method algorithms include MUltiple SIgnal Classification (MUSIC) and estimation of signal parameters via rotational invariant techniques (ESPRIT).

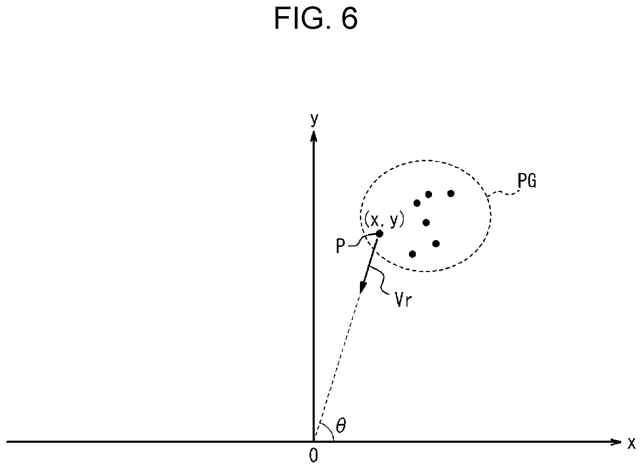

FIG. 6 illustrates an example of the result of the transformation of point group coordinates from the range-Doppler plane illustrated in FIG. 5 to the XY plane by the received signal processing unit 12 after performing direction estimation. The XY plane illustrated in FIG. 6 may be the same as the XY plane illustrated in FIG. 1. As illustrated in FIG. 6, the received signal processing unit 12 can plot a point group PG in the XY plane. The point group PG contains points P. Each of the points P has an angle θ and a radial velocity Vr in polar coordinates.

The received signal processing unit 12 detects an object present in the range where a transmission wave was transmitted, on the basis of at least one of the 2D-FFT or angle estimation results. The received signal processing unit 12 may perform object detection by performing, for example, clustering processing on the basis of respectively estimated information on distance, information on velocity, and angle information. Known algorithms used in clustering data include density-based spatial clustering of applications with noise (DBSCAN), for example. DBSCAN is an algorithm that performs density-based clustering. In the clustering processing, the average power of the points making up a detected object may be calculated, for example. The information on distance, information on velocity, angle information, and information on power pertaining to an object detected in the received signal processing unit 12 may be supplied to the external device 60 or the like via the communication interface 50, for example.

As above, the electronic device 100 may be provided with a transmitting antenna (transmitting antenna array 24), a receiving antenna (receiving antenna array 31), and the signal processing unit 10. The transmitting antenna array 24 transmits a transmission wave formed from a radio wave, for example. The receiving antenna array 31 receives a reflected wave resulting from the transmission wave being reflected. The signal processing unit 10 detects an object (an object such as the test subject 200, for example) that reflects the transmission wave, on the basis of the transmission signal transmitted as the transmission wave and the received signal received as the reflected wave.

The following further describes direction estimation of an arriving wave (an arriving reflected wave) by an antenna array of the electronic device 100 according to a comparative example of an embodiment.

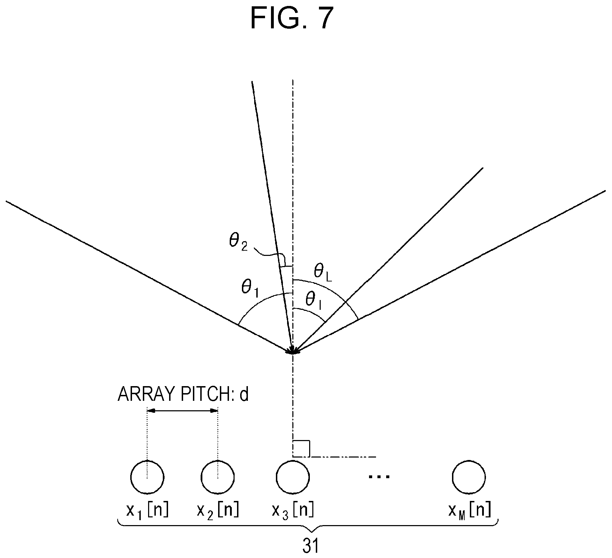

FIG. 7 is a diagram for describing the configuration of the receiving antenna array 31 and the principle of direction estimation of an arriving wave by the receiving antenna array 31 of the electronic device 100 according to a comparative example of an embodiment. FIG. 7 illustrates an example of the reception of radio waves by the receiving antenna array 31.

As illustrated in FIG. 7, the receiving antenna array 31 may be a linear arrangement of sensors such as receiving antennas. As illustrated in FIG. 7, in an embodiment, the receiving antenna array 31 may be configured to include a plurality of receiving antennas in a linear array. In FIG. 7, the plurality of antennas $x_1$, $x_2$, $x_3$, ..., $x_M$ that make up the receiving antenna array 31 are illustrated by small circles. The receiving antenna array 31 may include any plurality of antennas. As illustrated in FIG. 7, the plurality of antennas that make up the receiving antenna array 31 are spaced apart from one another by an array pitch d. This type of sensor array, in which sensors (such as antennas, ultrasonic transducers, and microphones) corresponding to various physical waves are disposed in an array, is also referred to as a uniform linear array (ULA). As illustrated in FIG. 7, the physical waves (such as electromagnetic waves and sonic waves) arrive from various directions, such as $\theta_1$ and $\theta_2$, for example. Herein, $\theta_1$ and $\theta_2$ may refer to the angle of arrival described above. In this way, a sensor array like the receiving antenna array 31 can estimate the direction of arrival (angle of arrival) by utilizing the phase difference that occurs in measurement values between sensors according to the direction of arrival of a physical wave. This type of technique for estimating the direction of arrival of a wave is also referred to as angle-of-arrival estimation or direction-of-arrival (DoA) estimation.

In the electronic device 100 according to a comparative example of an embodiment, at least one of the transmitting antenna array 24 or the receiving antenna array 31 may have a plurality of antennas in a linear arrangement. This allows for appropriate narrowing of directivity in the transmission and reception of radio waves in a millimeter-wave radar, for example. When transmitting a transmission wave, the direction of the transmission beam is often controlled by a beamformer. On the other hand, when receiving a reflected wave, the direction of arrival of the reflected wave is more often estimated by subspace methods (such as MUSIC and ESPRIT described above) than by a beamformer. With beamformers and subspace methods, for electromagnetic waves arriving from various directions in the ULA as illustrated in FIG.

7, a phase difference occurs in measurement values between sensors depending on the direction of arrival. Accordingly, this phase difference can be utilized to estimate the direction of arrival of a reflected wave.

The following further describes angle estimation in two directions of an arriving wave by an antenna array of the electronic device 100 according to a comparative example of an embodiment.

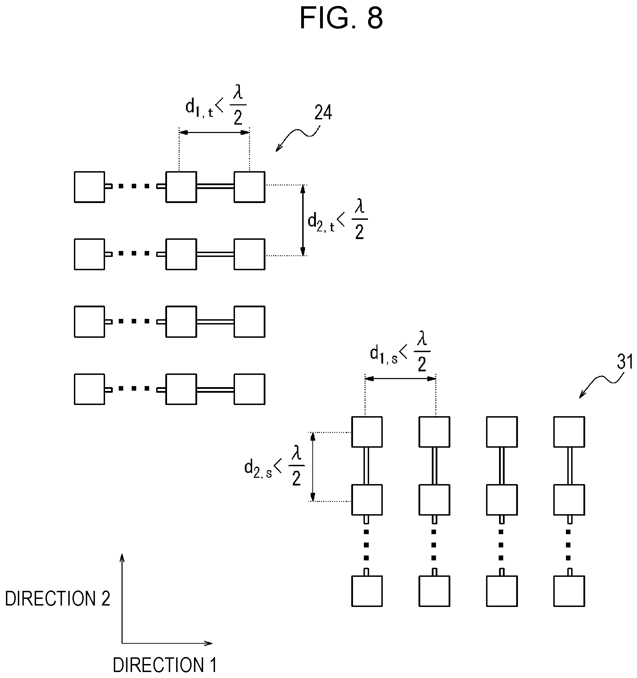

FIG. 8 illustrates an example arrangement of antennas for estimating the direction of arrival with respect to two orthogonal angles.

As illustrated in FIG. 8, in the electronic device 100 according to a comparative example of an embodiment, the transmitting antenna array 24 and/or receiving antenna array 31 may be configured to include an array of a plurality of patch antenna units.

In the transmitting antenna array 24 illustrated in FIG. 8, one patch antenna unit may be configured to include a plurality of elements electrically connected in the direction of direction 1 illustrated in the diagram. In each of the patch antenna units, the plurality of elements may be electrically connected by wiring such as striplines on a board, for example. In each of the patch antenna units, the plurality of elements may be spaced apart from one another by a pitch $d_{1,t}$ shorter than half the wavelength $\lambda$ of the transmission wave. In FIG. 8, each of the patch antenna units may have any number of two or more elements electrically connected.

As illustrated in FIG. 8, the transmitting antenna array 24 may include a plurality of patch antenna units arrayed in the direction of direction 2 illustrated in the diagram. The patch antenna units may be spaced apart from one another by a pitch $d_{2,t}$ shorter than half the wavelength $\lambda$ of the transmission wave. In an embodiment, the transmitting antenna array 24 may include any number of two or more patch antenna units.

As illustrated in FIG. 8, in an embodiment, the receiving antenna array 31 may be a variation of the arrangement of the plurality of elements in the transmitting antenna array 24. That is, in the receiving antenna array 31 illustrated in FIG. 8, one patch antenna unit may be configured to include a plurality of elements electrically connected in the direction of direction 2 illustrated in the diagram. In each of the patch antenna units, the plurality of elements may be electrically connected by wiring such as striplines on a board, for example. In each of the patch antenna units, the plurality of elements may be spaced apart from one another by a pitch $d_{2,s}$ shorter than half the wavelength $\lambda$ of the transmission wave. In FIG. 8, each of the patch antenna units may have any number of two or more elements electrically connected.

As illustrated in FIG. 8, the receiving antenna array 31 may include a plurality of patch antenna units arrayed in the direction of direction 1 illustrated in the diagram. The patch antenna units may be spaced apart from one another by a pitch $d_{1,s}$ shorter than half the wavelength $\lambda$ of the transmission wave. In an embodiment, the receiving antenna array 31 may include any number of two or more patch antenna units.

The elements included in the transmitting antenna array 24 and the receiving antenna array 31 may all be arranged in the same plane (on the surface layer of the same board, for example). The transmitting antenna array 24 and the receiving antenna array 31 may also be arranged in proximity to each other (monostatic). Direction 1 and direction 2 illustrated in FIG. 8 may be geometrically orthogonal.

With the transmitting antenna array 24 and the receiving antenna array 31 as illustrated in FIG. 8, the directivity of each of the transmitting antenna and the receiving antenna can be narrowed appropriately. By using the transmitting antenna array 24 as illustrated in FIG. 8 to control the direction in which to transmit each transmission wave at each timing for transmitting a transmission wave (transmission signal), a beamformer can be realized for the direction of direction 2 illustrated in FIG. 8. By using the receiving antenna array 31 as illustrated in FIG. 8, estimation of the direction of arrival of the reflected wave can be realized for the direction of direction 1 illustrated in FIG. 8. This enables estimation of the direction of arrival of a reflected wave with respect to two substantially orthogonal angles. Accordingly, a point group indicating an object such as the test subject 200 can be acquired three-dimensionally.

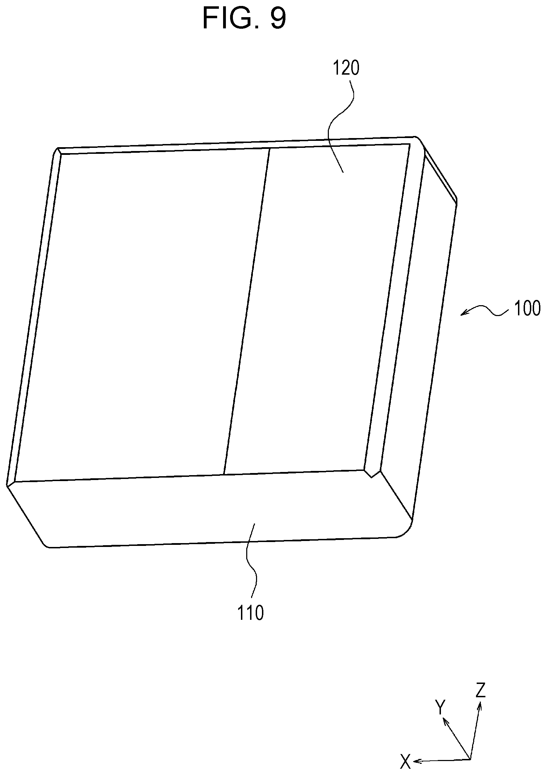

FIG. 9 is a diagram illustrating the exterior of the electronic device 100 according to a comparative example of an embodiment.

As illustrated in FIG. 9, in a comparative example of an embodiment, the electronic device 100 is provided with an enclosure 110. The enclosure 110 may be formed from resin such as plastic, or may be formed from metal, for example. The enclosure 110 may function to protect the internal structure of the electronic device 100. At least a part of the enclosure 110 may be configured as a radome unit 120. At least a part of the radome unit 120 may be formed from a material that is transmissive with respect to a transmission wave transmitted from the electronic device 100 and transmissive with respect to a received wave (reflected wave) received by the electronic device 100.

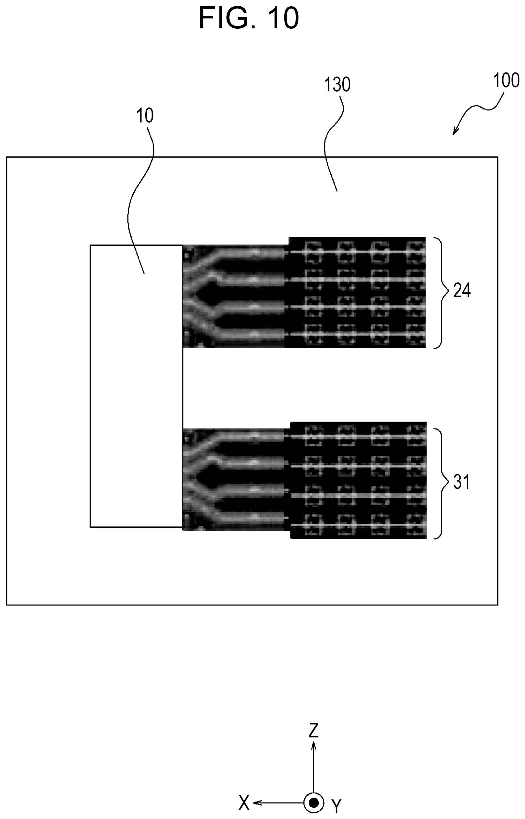

FIG. 10 is a diagram illustrating an example of the structure inside the enclosure of the electronic device 100 according to a comparative example of an embodiment.

As illustrated in FIG. 10, in a comparative example of an embodiment, the electronic device 100 may be configured to have an internal structure including a board 130. The transmitting antenna array 24, the receiving antenna array 31, and the signal processing unit 10 may be disposed on the board 130, for example. The transmission DAC 21, the transmission circuit 22, the millimeter-wave transmission circuit 23, and the like illustrated in FIG. 2 may also be disposed on the board 130, as appropriate. The mixer 32, the reception circuit 33, the reception ADC 34, and the like illustrated in FIG. 2 may also be disposed on the board 130, as appropriate. Components described above such as the amplifier

EP 4 772 903 A1

(amplification circuit) that amplifies the power (electrical energy) of the transmission wave (transmission signal) and/or the low-noise amplifier (LNA) that amplifies the reflected wave (received signal) that the receiving antenna array 31 receives may also be disposed on the board 130.

For example, in the electronic device 100, most of the transmission DAC 21, the transmission circuit 22, the millimeter-wave transmission circuit 23, the mixer 32, the reception circuit 33, the reception ADC 34, and the signal processing unit 10 may be disposed on the board 130. In this case, these functional units may be implemented in a radar circuit unit (system on a chip (SoC)) centered on a monolithic microwave integrated circuit (MMIC). The MMIC/SoC described above may be implemented as the signal processing unit 10 on the board 130 illustrated in FIG. 10, for example. The MMIC/SoC may be connected to the transmitting antenna array 24 and the receiving antenna array 31 disposed on the board 130 through interlayer vias, microstrip lines, or coplanar lines in a multilayer board.

FIG. 10 illustrates an example of an antenna with four channels for transmission and reception. In the configuration example illustrated in FIG. 10, the MMIC/SoC, the transmitting antenna array 24, and the receiving antenna array 31 are mounted on the board 130. In this case, the board 130 may be integrally housed in the enclosure 110, together with a heat dissipation mechanism and/or a mechanical component for ensuring rigidity. In the present disclosure, a channel of a transmitting antenna or a receiving antenna may be an antenna formed from a single antenna element or a combination of antenna elements for transmitting a transmission signal or receiving a reception signal. For example, if there are n transmitting antennas formed from m transmitting antenna elements, where m and n are any given integers, the antenna may be considered to be an n-channel transmitting antenna. As another example, if there are n receiving antennas formed from m receiving antenna elements, where m and n any given integers, the antenna may be considered to be an n-channel receiving antenna.

Concerns like the following may be applicable to the electronic device 100 according to a comparative example of an embodiment described above.

In the case of detecting biological information such as the heartbeat of a user by transmitting and receiving radio waves, it may be necessary to control the directivity of the transmission wave and/or perform direction estimation processing on the received wave by using a sufficiently abundant number of transmitting and receiving antennas. In such a case, it is desirable to use an antenna with four or more channels for both transmission and reception. However, it is not necessarily easy to reduce the overall size by integrating the transmitting antenna array 24 and the receiving antenna array 31 together with the signal processing unit 10 that includes the MMIC/SoC.

In general, adequately and properly executing processing for a radar sensor as illustrated in FIG. 10 means that a CPU/DSP or other processor in a circuit unit (the signal processing unit 10) such as an SoC tends to generate more heat. Therefore, it is assumed that it may be necessary to increase the size of, for example, the heat dissipation mechanism in the electronic device 100 according to a comparative example of an embodiment.

Considering the above concerns, it is also assumed that an integrated configuration such as the enclosure 110 illustrated in Figure 9 for example may not be feasible in situations where the configuration is to be mounted in a narrow space, such as in the case of sensing the vitals of a user inside the cabin of an automobile.

Another concern is that, given a radar sensor configuration like the one illustrated in FIG. 10, the area that is detectable by a single MMIC/SoC (the signal processing unit 10) may be limited. As an example, assume that a millimeter-wave radar is used to detect a micro-Doppler signature and thereby detect chest vibrations associated with the heartbeat of a user or slight vibrations in another object such as a machine or a building. In this case, a certain angular range (about ±30°, for example) at the front face of the millimeter-wave sensor is the field of view (FOV) of the radar. Consequently, using a single MMIC/SoC (signal processing unit 10) may be problematic in that only a specific narrow area can be detected.

Accordingly, through various research and demonstration experiments, the applicant has arrived at an electronic device according to an embodiment as a configuration that may address issues like the above. The following describes such an electronic device according to an embodiment.

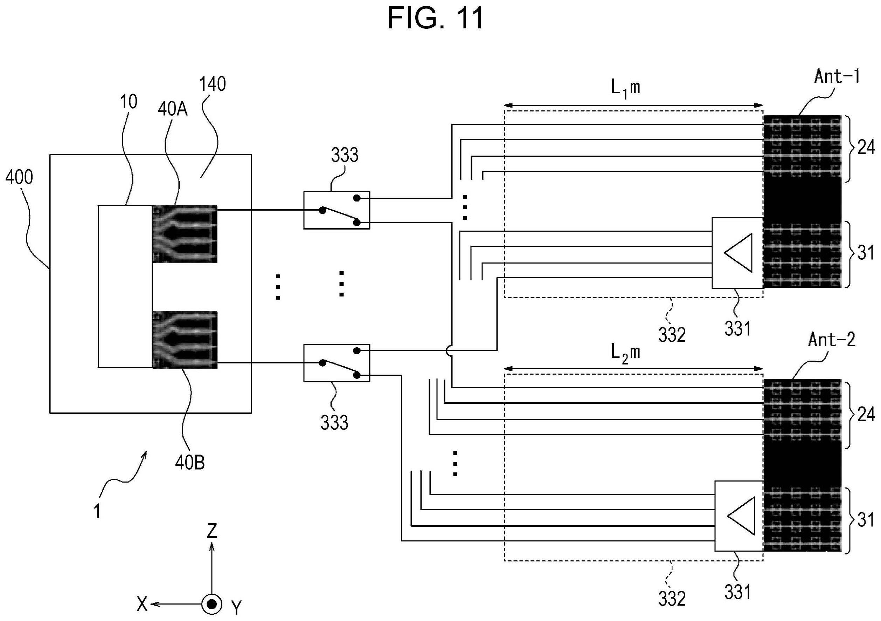

FIG. 11 is a diagram illustrating an example of the schematic structure of an electronic device according to an embodiment. In the electronic device 1 according to an embodiment illustrated in FIG. 11, the description will be simplified or omitted, as appropriate, for functional units that are the same and/or similar to the electronic device 100 according to a comparative example of an embodiment.

As illustrated in FIG. 11, in an embodiment, the electronic device 1 may be provided with a plurality of antenna units, such as Ant-1 and Ant-2, for example. The electronic device 1 may be provided with a circuit unit 400 including a board 140. The circuit unit 400 may include the signal processing unit 10. In FIG. 11, in an embodiment, the electronic device 1 may be provided with two antenna units referred to as Ant-1 and Ant-2. In an embodiment, the electronic device 1 may be provided with any number of at least one antenna unit Ant. On the other hand, as described later, in an embodiment, the electronic device 1 may also be provided with only one antenna unit, such as only Ant-1, for example.

As illustrated in FIG. 11, the antenna unit Ant-1 may be provided with a pattern of an antenna array (antenna pattern). For example, the antenna unit Ant-1 may be a rigid-substrate antenna or a flexible antenna connected by microstrip lines or coplanar lines mounted on a flexible substrate, for example. The antenna unit Ant-1 may be provided with the transmitting antenna array 24 and the receiving antenna array 31. As illustrated in FIG. 11, the antenna unit Ant-1 may be provided with multiple transmitting antennas array 24 and multiple receiving antenna arrays 31. In FIG. 11, the antenna unit Ant-1 is provided with four transmitting antennas array 24 and four receiving antenna arrays 31. However, the antenna unit Ant-1 may include any number of transmitting antenna arrays 24 and any number of receiving antenna arrays 31.

The transmitting antenna array 24 and the receiving antenna array 31 are each electrically connected to an RF switch 333 via a transfer line part 332. The transmitting antenna array 24 may be electrically connected to the transfer line part 332. The receiving antenna array 31 may be electrically connected to the transfer line part 332 via a low-noise amplifier (LNA) 331. That is, the signal received by the receiving antenna array 31, the LNA 331 may be a low-noise amplifier that amplifies received radio waves while maintaining low noise. In amplifying a received signal, a low-noise amplifier will have a better S/N ratio when the low-noise amplifier is located closer to the receiving antenna. Consequently, the LNA 331 may be connected in the vicinity of the receiving antenna array 31. The received signal may be amplified while maintaining low noise by the A 331, and then supplied to the transfer line part 332. The electronic device 1 may also be provided with an amplifier for amplifying the transmission signal at any position before the transmission signal is transmitted from the transmitting antenna array 24. The RF switch 333 connected to the transfer line part 332 may be connected to the signal processing unit 10. The signal processing unit 10 may be implemented by the MMIC/SoC described above.

As illustrated in FIG. 11, the transfer line part 332 connected to the antenna unit Ant-1 may have a length $L_1$ [m]. The length of the transfer line part 332 connected to the antenna unit Ant-1 may be set as appropriate (such as from a few centimeters to a few meters, for example) according to the purpose or the like of the electronic device 1.

The antenna unit Ant-2 may have the same and/or similar configuration to the antenna unit Ant-1. Like the antenna unit Ant-1, the antenna unit Ant-2 may be connected to the signal processing unit 10 via an RF switch 333. In the case of providing another antenna unit Ant other than the antenna unit Ant-1 and the antenna unit Ant-2, the other antenna unit Ant may have the same and/or similar configuration to the antenna unit Ant-1 and the antenna unit Ant-2.

The antenna unit Ant-1, the antenna unit Ant-2, and/or the like may include patterns of transmitting and receiving antennas corresponding to respective antenna sets. The antenna unit Ant-1, the antenna unit Ant-2, and/or the like may be mounted by using a rigid dielectric substrate or a ceramic substrate, or by using a flexible dielectric substrate.

As illustrated in FIG. 11, the transfer line part 332 connected to the antenna unit Ant-2 may have a length $L_2$ [m]. The length of the transfer line part 332 connected to the antenna unit Ant-2 may be set as appropriate (such as from a few centimeters to a few meters, for example) according to the purpose or the like of the electronic device 1.

The transfer line part 332 may be any of various types of members for conducting radio waves to respective transmitting and receiving antenna sets. For example, the transfer line part 332 may be formed using a coaxial cable, a flexible cable, and/or the like. The transfer line part 332 may also be microstrip lines or coplanar lines mounted on a flexible dielectric substrate, for example.

When implemented as hardware, the transfer line part 332 may be laid out so as to be as equal in length as possible for all antenna channels.

The RF switch 333 may function to switch each of the plurality of transmitting antenna arrays 24 and receiving antenna arrays 31. The RF switch 333 may be a highfrequency switch such as a single-pole double-throw (SPDT) switch or a single-pole n-throw (SPnT) switch (where n is a natural number equal to or greater than 2). In an embodiment, the RF switch 333 may be mounted on the board 140 included in the circuit unit 400, for example. The number of RF switches 333 depends on the value of n in the case of a single-pole n-throw (SPnT) switch (where n is a natural number equal to or greater than 2), and the number Sn of transmitting antenna arrays 24 and the number Rn of receiving antenna arrays 31 in each channel. For example, if n = 2, Sn = 2, and Rn = 2, there may be two RF switches 333. The RF switch 333 may also be a p-pole q-throw switch, where each of p and q is any given integer. In the case illustrated in FIG. 11, Sn = 4 and Rn = 4, and therefore the number of single-pole double-throw (SPDT) RF switches 333 is four for the transmitting antenna array 24 and four for the receiving antenna array 31, for a total of eight.

As illustrated in FIG. 11, in the electronic device 1, a waveguide formed by the transfer line part 332 is extended from the signal processing unit 10 to the antenna unit Ant-1 and/or the like. FIG. 11 conceptually illustrates a waveguide 40A and a waveguide 40B leading from the signal processing unit 10 to the RF switches 333. An RF switch 333 is provided with respect to each waveguide extended by the transfer line part 332. Each RF switch 333 may be a single-pole double-throw (SPDT) switch or a single-pole n-throw (SPnT) switch to suit the number of transmitting/receiving antenna sets connected thereto. FIG. 11 illustrates an example in which each RF switch 333 connects two transmitting/receiving antenna sets.

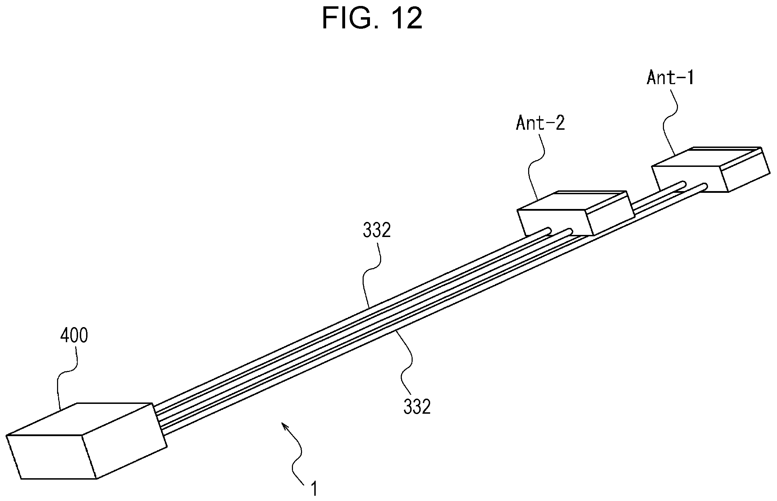

FIG. 12 is a diagram schematically illustrating an example of the exterior of the electronic device 1 illustrated in FIG. 11. FIG. 12 illustrates a situation in which the configuration illustrated in FIG. 11 is housed inside an enclosure, for example.

When implemented as hardware, the electronic device 1 may be provided with a circuit unit 400 and antenna units Ant-1, Ant-2, and/or the like separate from the circuit unit 400, as illustrated in FIG. 12. The circuit unit 400 and the antenna units Ant-1, Ant-2, and/or the like may be electrically connected to each other by the transfer line part 332. The circuit unit 400 may include the signal processing unit 10 (and the board 140) and/or the like illustrated in FIG. 12, for example. In FIG. 12, the transfer line part 332 may be a coaxial cable. The transfer line part 332 may also be a flexible cable or some other kind of wiring.

As illustrated in FIG. 12, the electronic device 1 may be provided with a plurality of antennas units, such as the antenna unit Ant-1, the antenna unit Ant-2, and so on. Accordingly, in the electronic device 1, the plurality of antenna units such as the antenna unit Ant-1, the antenna unit Ant-2, and so on can be mounted in respectively different places.

Such a configuration enables the electronic device 1 to appropriately sense the heartbeat/respiration/body movement and/or the like of a human being or other entity, even in cases requiring a compact implementation of the radar sensor or the like. For example, if the electronic device 1 as illustrated in FIG. 12 is applied to an environment such as the interior of an automobile, the electronic device 1 can sense the heartbeat/respiration/body movement and/or the like of a driver, for example. In this case, the circuit unit 400 of the electronic device 1 may be mounted near an in-vehicle ECU of the automobile, for example. The antenna unit Ant-1 of the electronic device 1 may be mounted on the front face of a steering wheel of the automobile. The antenna unit Ant-2 of the electronic device 1 may be mounted near a driving mirror (rear-view mirror) of the automobile.

Such an implementation allows for the electronic device 1 to robustly detect biological information and/or the like about the driver even if the body of the driver shifts position, for example, while also allowing for a space-saving implementation.

The following describes operations by the electronic device 1 according to an embodiment.

To appropriately carry out detection by the electronic device 1 as illustrated in FIGs. 11 and 12, in an embodiment, the electronic device 1 may execute various correction operations through digital processing, for example. The following further describes various correction operations to be executed by the electronic device 1 according to an embodiment.

(1. Correction accounting for electrical length of transfer line part)

As illustrated in FIGs. 11 and 12, in the electronic device 1 according to an embodiment, the antenna unit Ant-1 and the antenna unit Ant-2 are extended via the transfer line part 332. If the electrical length over which radio waves are transmitted and received is extended via a transfer line part in this way, the range (distance) over which the radar sensor detects is shifted toward a longer distance by an amount corresponding to the extended electrical length. Consequently, in an embodiment, the electronic device 1 may apply correction that accounts for the electrical length of transmission and reception extended by the transfer line part 332.

In general, electromagnetic waves propagating within a medium such as a coaxial cable, a microstrip line, or a coplanar line undergo wavelength shortening due to the dielectric substance constituting the medium. For example, in the case where the transfer line part 332 is a coaxial cable, with L [m] denoting the coaxial cable length and $\varepsilon'_r$ denoting the real part of the relative permittivity of the dielectric substance of the coaxial cable, the wavelength shortening due to the dielectric substance can be expressed as in the following expression (1).
[Math. 1]

$$L' = \frac{L}{\sqrt{\epsilon'_r}} \qquad (1)$$

Consequently, in the electronic device 1, when the electrical length of transmission and reception extended by the transfer line part 332 is accounted for, the resulting length is equivalent to the radio waves having propagated a distance of L' [m] indicated in expression (1) above. In this way, the length of wiring from device to device in an electronic circuit may be expressed not by the length in a physical sense (physical length) but in terms of signal delay time, also referred to as electrical length.

The correction described above may need to be applied to a value involved in performing a range FFT in situations where, for example, a 2D-FFT is executed in the signal processing unit 10 illustrated in FIG. 2. That is, provided that d [m] denotes the range resolution of the 2D-FFT and r denotes the calculated value of the range bins of the 2D-FFT, a corrected value r' can be expressed as in the following expression (2).
[Math. 2]

$$r' = r - \text{round}\left(\frac{2L'}{d}\right) \qquad (2)$$

In expression (2) above, round is a function representing processing for rounding to the decimal point. The numerator of the fraction within the parentheses of the round function in expression (2) above is multiplied by 2 as a coefficient. This accounts for the round-trip path on which the transfer line part is of equal length for transmission and reception.

(2. Correction accounting for phase between channels)

If the transfer line part 332 is extended by a relatively long cable, phase delay differences between channels will occur due to the design, regardless of how all channels are wired to be of equal length. For this reason, deviations may occur in the direction-of-arrival (DOA) estimation. Consequently, in an embodiment, the electronic device 1 may correct such deviations.

(3. Correction accounting for electromagnetic wave loss)

If the transfer line part 332 is extended, the electromagnetic wave loss as seen from the final stage of the transmission/reception circuit in the electronic device 1 changes compared to the case where the transfer line part 332 is not extended. For this reason, the loss may deviate from the loss according to the radar equation. If such deviations occur, correction may be necessary when performing signal processing based on an assumed human radar cross-section (RCS), for example. Consequently, in an embodiment, the electronic device 1 may apply such a correction.

The following further describes "2. Correction accounting for phase between channels" and "3. Correction accounting for electromagnetic wave loss" indicated above.

As an example, assume that in the electronic device 1 according to an embodiment, the transfer line part 332 is provided with respect to each of the antenna unit Ant-1 and the antenna unit Ant-2, and the line lengths thereof are slightly different. For example, assume that out of the transfer line part 332 used to extend the antenna unit Ant-1 and the transfer line part 332 used to extend the antenna unit Ant-2, one is 60 cm and the other is 61 cm. The following examines the amplitude and the phase between the antenna patches in the antenna units Ant for each of the channels and the signal processing unit 10 (MMIC) in the electronic device 1 according to an embodiment configured as above.

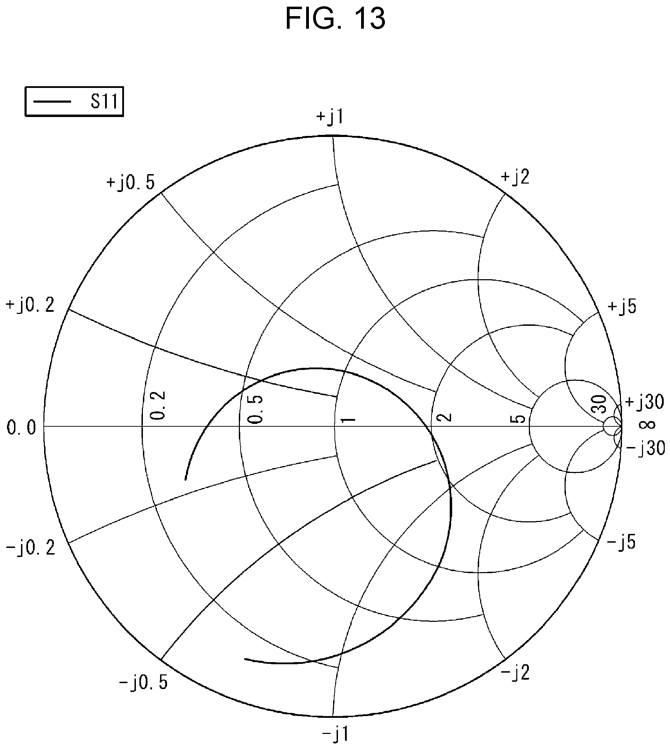

FIG. 13 is a diagram illustrating an example of a Smith chart of the scattering parameter (S-parameter) $S_{11}$ in a configuration that does not include the transfer line part 332, as in the case of the electronic device 100 according to a comparative example of an embodiment illustrated in FIG. 10. FIG. 13 illustrates a Smith chart of $S_{11}$ from 60 GHz to 67 GHz.

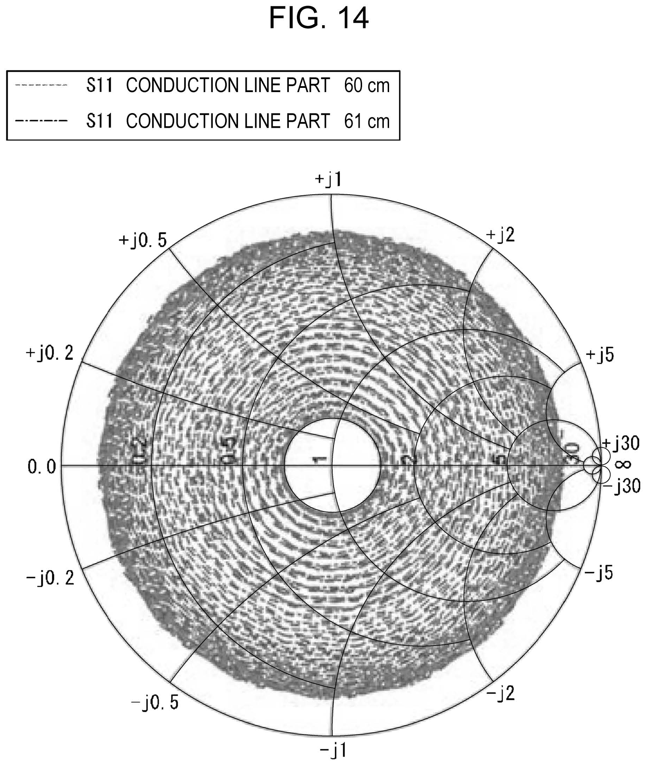

FIG. 14 is a diagram illustrating an example of a Smith chart of the scattering parameter (S-parameter) $S_{11}$ in a configuration that includes the transfer line part 332, as in the case of the electronic device 1 according to an embodiment illustrated in FIG. 11. FIG. 14 illustrates a Smith chart of $S_{11}$ from 60 GHz to 67 GHz. The following assumes that out of the transfer line part 332 used to extend the antenna unit Ant-1 and the transfer line part 332 used to extend the antenna unit Ant-2, one is 60 cm and the other is 61 cm. The following also assumes that the transfer line part 332 is a coaxial cable in which the outer conductor radius is 2.6 mm, the inner conductor radius is 0.7 mm, and the dielectric layer is polyethylene. FIG. 14 illustrates $S_{11}$ for the 60 cm transfer line part 332 and $S_{11}$ for the 61 cm transfer line part 332.

A comparison of FIGs. 13 and 14 reveals that if the transfer line part 332 is included, as in the case of the electronic device 1 according to an embodiment, the phase rotates through many revolutions on the Smith chart (FIG. 14).

The following indicates the amplitude and the phase between an antenna patch for one channel and the signal processing unit 10 (MMIC).

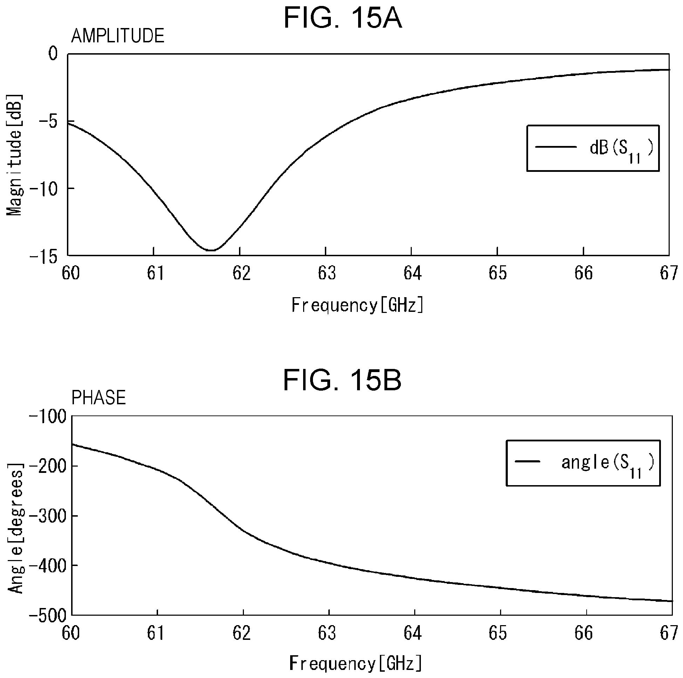

FIGs. 15A and 15B are diagrams illustrating an example of the transfer loss and the phase delay of $S_{11}$ in a configuration that does not include the transfer line part 332, as in the case of the electronic device 100 according to a comparative example of an embodiment illustrated in FIG. 10. FIG. 15A illustrates the change in amplitude (in units of dB) with respect to frequency. FIG. 15B illustrates the change in phase (in units of degrees) with respect to frequency. FIGs. 15A and 15B illustrate $S_{11}$ from 60 GHz to 67 GHz. In the electronic device 100 according to a comparative example of an embodiment, the antenna for one channel is designed to resonate at 61.5 GHz. As illustrated in FIG. 15A, resonance at 61.5 GHz can be confirmed.

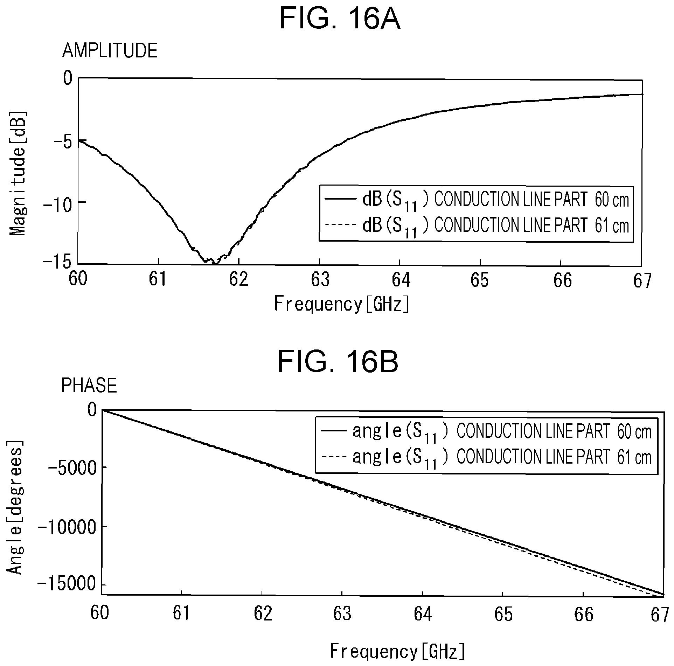

FIGs. 16A and 16B are diagrams illustrating an example of the transfer loss and the phase delay of $S_{11}$ in a configuration that includes the transfer line part 332, as in the case of the electronic device 1 according to an embodiment illustrated in FIG. 11. FIG. 16A illustrates the change in amplitude (in units of dB) with respect to frequency. FIG. 16B illustrates the change in phase (in units of degrees) with respect to frequency. FIGs. 16A and 16B illustrate $S_{11}$ from 60 GHz to 67 GHz. FIGs. 16A and 16B illustrate $S_{11}$ for the 60 cm transfer line part 332 and $S_{11}$ for the 61 cm transfer line part 332.

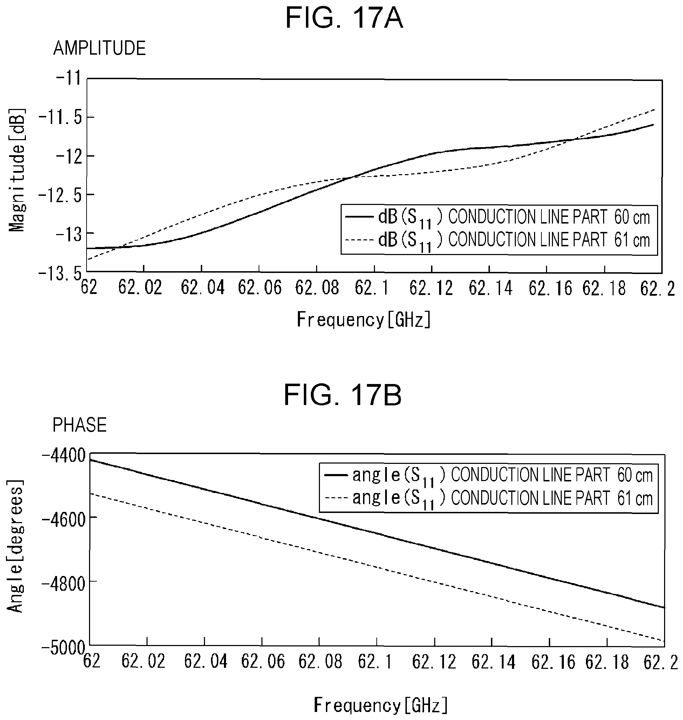

FIGs. 17A and 17B are diagrams illustrating a partially enlarged view of FIGs. 16A and 16B, respectively. More specifically, FIGs. 17A and 17B illustrate an enlarged view of the amplitude and the phase in the range from 62 GHz to 62.2 GHz (a 200 MHz span) in FIGs. 16A and 16B, respectively.

As illustrated in FIGs. 16A and 16B, although the amplitude and the phase of $S_{11}$ overall do not greatly differ from the amplitude and the phase illustrated in FIGs. 15A and 15B, small fluctuations are present. As FIGs. 17A and 17B demonstrate, changes in both the amplitude and the phase can be seen even with a difference of just 1 cm, such as between 60 cm and 61 cm, in the line lengths of the transfer line part 332. As illustrated in FIGs. 17A and 17B, a difference of 1 cm in the line lengths of the transfer line part 332 results in an amplitude difference of approximately 0.5 dB and a phase difference of approximately 100 degrees.

To correct changes like those described above, the electronic device 1 according to an embodiment may designate a certain reference channel and measure the phase difference between the reference channel and another channel using a network analyzer or the like. Through such measurement, the electronic device 1 according to an embodiment can correct the amplitude difference and/or the phase difference described above.

More specifically, correction may be applied as follows. In the electronic device 1 according to an embodiment, the signal processing unit 10 defines $N_t$ to be the number of measurement data transmission channels, and defines $N_r$ to be the number of measurement data reception channels. The signal processing unit 10 may correct the amplitude and the phase of radio waves transmitted and received by each channel, using a first channel as a reference. The signal processing unit 10 may apply the correction of the amplitude and the phase as described above as processing to be performed after a reflected wave resulting from a transmission wave being reflected is received and before processing such as the 2D-FFT is performed.

For the i-th reception channel, the signal processing unit 10 defines $\Delta a_i^r$ to be an amplitude correction value and $\Delta \varphi_i^r$ to be a phase correction value. For the j-th transmission channel, the signal processing unit 10 defines $\Delta a_j^t$ to be an amplitude correction value and $\Delta \varphi_j^t$ to be a phase correction value. In an embodiment, the signal processing unit 10 may store these correction values in any memory, for example.

The signal processing unit 10 may perform the calculation processing indicated in expressions (3) and (4) below with respect to the amplitude $a_i^r$ and the phase $\varphi_i^r$ of an actual received signal and the amplitude $a_j^{tr}$ and the phase $\varphi_j^t$ of an actual transmission signal. In this way, the signal processing unit 10 can calculate a corrected i-th received signal vector $s_i^r$ and a corrected j-th transmission signal vector $s_j^t$. In expressions (3) and (4), i is a number starting from 2, and j is a number starting from 2.

[Math. 3]

$$s_i^r = (a_i^r + \Delta a_i^r)\, e^{\phi_i^r + \Delta \phi_i^r}\,, i = 2, \cdots, N_r \qquad (3)$$

[Math. 4]

$$s_j^t = \left(a_j^t + \Delta a_j^t\right) e^{\phi_j^t + \Delta \phi_j^t}\,, j = 2, \cdots, N_t \qquad (4)$$

The following further describes a specific processing method using expressions (3) and (4) above.

An example of the amplitude and the phase at the transmitting and receiving ends of the transmitting antenna array 24 and the receiving antenna array 31 of an antenna unit Ant is as illustrated in FIG. 17. On the other hand, correction using expressions (3) and (4) above may be executed by digital signal processing in the signal processing unit 10, for example. In the case of applying this correction to processing for detecting (estimating) heartbeat, for example, amplitude correction and phase correction of a received signal may be performed before basic radar processing such as 2D-FFT processing, CFAR processing, and DoA processing.

In an embodiment, the signal processing unit 10 may apply amplitude and phase correction using a certain channel as a reference on an antenna that is to transmit or receive radio waves in an antenna unit Ant. In this case, the signal processing unit 10 measures the difference in the amplitude and the phase of $S_{11}$ as seen from the board edge of the board 140 illustrated in FIG. 11 for the sensor system of an electronic device 1 that has been designed or actually fabricated.

In this way, the signal processing unit 10 can obtain the amplitude correction value $\Delta a_i^r$ and the phase correction value $\Delta \varphi_i^r$ for the i-th reception channel and the amplitude correction value $\Delta a_j^t$ and the phase correction value $\Delta \varphi_j^t$ for the j-th transmission channel. Consequently, the signal processing unit 10 can apply correction according to expressions (3) and (4) above. These correction values, namely $\Delta a_i^r$, $\Delta \varphi_i^r$, $\Delta a_j^t$, and $\Delta \varphi_j^t$, may be stored in advance in a memory of the radar SoC. The signal processing unit 10 may read out the correction values stored in the memory every time the signal processing unit 10 is to perform processing for transmitting and receiving radio waves.

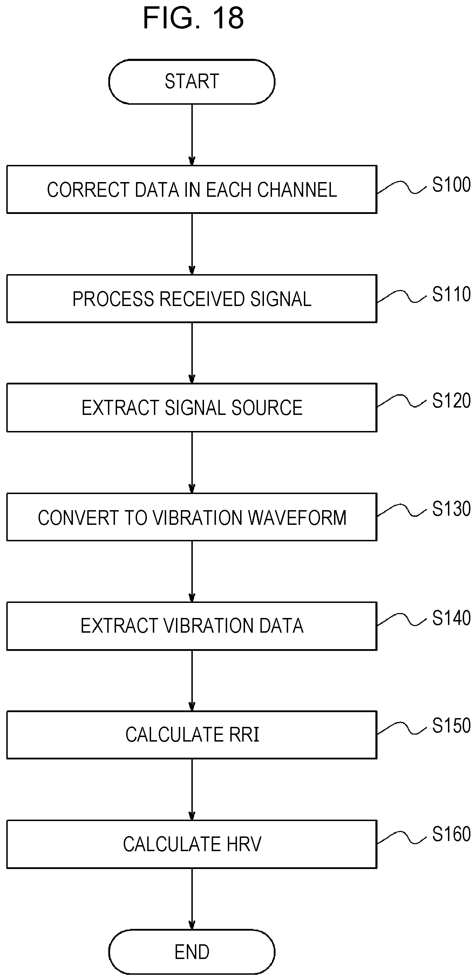

FIG. 18 is a flowchart illustrating an example of heartbeat interval estimation operations by the electronic device 1 according to an embodiment. The following refers to FIG. 18 to schematically describe heartbeat interval estimation operations by the electronic device 1 according to an embodiment.

FIG. 18 illustrates operations after the electronic device 1 according to an embodiment has received a reflected wave. That is, the operations illustrated in FIG. 18 presuppose that the electronic device 1 illustrated in FIG. 11 has transmitted a transmission wave (transmission signal) from the transmitting antenna array 24. At least a portion of the transmission wave transmitted from the electronic device 1 is reflected by the test subject 200 (the chest area thereof, for example) to become a reflected wave. The electronic device 1 illustrated in FIG. 11 receives such a reflected wave from the receiving antenna array 31 and converts the reflected wave into a digital signal (reception ADC 34) (see FIG. 2). From that point, the operations illustrated in FIG. 18 begin.

When the operations illustrated in FIG. 18 begin, in step S100, the signal processing unit 10 of the electronic device 1 corrects the data in each channel of a plurality of antenna units Ant, such as the antenna unit Ant-1 and the antenna unit Ant-2, for example. The correction applied at this point may be "2. Correction accounting for phase between channels" and/or "3. Correction accounting for electromagnetic wave loss". That it, in step S100, the signal processing unit 10 may apply amplitude correction and phase correction to the received signal. The correction applied at this point may also include "1. Correction accounting for electrical length of transfer line part". Such correction operations may be performed by the received signal processing unit 12 of the signal processing unit 10, for example.

In step S110, the signal processing unit 10 processes the signal that has been received (received signal). The signal processing performed in step S110 may include the 2D-FFT, CFAR processing, and/or direction-of-arrival estimation described above, for example. Such operations may be performed by the received signal processing unit 12 of the signal processing unit 10, for example.

In step S120, the signal processing unit 10 extracts a signal source from the information processed in step S110. The signal processing performed in step S120 may include processing for filtering the data that is the result of performing the 2D-FFT processing, for example. In step S120, the signal processing unit 10 may extract a spectral component at only the position where the test subject 200 is present. The position where the test subject 200 is present may be identified by any of various known techniques. Such operations may be performed by the heartbeat extraction unit 13 of the signal processing unit 10, for example.

In step S130, the signal processing unit 10 converts the processing result of the previous stage into a vibration waveform. The processing performed in step S130 may include processing to extract phase information from IQ data, for example. In step S130, the signal processing unit 10 may include processing to extract vibration data including heart sound from the spectral component of the 2D-FFT processing of the test subject 200 extracted in step S120. Such operations may be performed by the heartbeat extraction unit 13 of the signal processing unit 10, for example.

In step S140, the signal processing unit 10 extracts vibration data from the processing result of the previous stage. The processing performed in step S140 may include frequency filtering, for example. In step S140, the signal processing unit 10 may perform frequency filtering to extract a low-frequency signal including the envelope curve of the heart sound of the test subject 200. Such operations may be performed by the heartbeat extraction unit 13 of the signal processing unit 10, for example.

In step S150, the signal processing unit 10 calculates the RR interval (RRI) of the test subject 200 by detecting peaks in the beating of the test subject 200 from the processing result of the previous stage and calculating the interval between the peaks. In step S150, the signal processing unit 10 may detect peaks in the low-frequency signal including the envelope curve of the heart sound of the test subject 200. In step S150, the signal processing unit 10 may also calculate and/or extract the interval between peaks. Such operations may be performed by the calculation unit 14 of the signal processing unit 10, for example. As above, in step S150, the signal processing unit 10 may extract the heart sound interval.

In step S160, the signal processing unit 10 may calculate the heart rate variability (HRV) of the test subject 200. In step S160, the signal processing unit 10 may calculate the HRV of the test subject 200 by calculating the spectral density of RRI time-series data. Calculating the power spectral density of RRI time-series data may involve using Welch's method or the like to perform frequency analysis on an RRI time-series waveform. Such operations may be performed by the calculation unit 14 of the signal processing unit 10, for example. In step S160, the signal processing unit 10 may also analyze the spectrum from the processing result of the previous stage.

In this way, in an embodiment, the electronic device 1 is provided with: an antenna unit Ant that includes a plurality of antenna elements; and a signal processing unit 10 that generates a transmission signal to be transmitted from the antenna unit Ant and processes a received signal that the antenna unit Ant receives. In an embodiment, the electronic device 1 is provided with: a transmission antenna unit Ant that has a plurality of transmission channels; and a transmission signal processing unit 10 that generates a transmission signal to be transmitted from the transmission antenna unit Ant. The electronic device 1 may be provided with: a reception antenna unit Ant that has a plurality of reception channels; and a reception signal processing unit 10 that processes a received signal received from the reception antenna unit Ant.

In an embodiment, the electronic device 1 may be provided with a transfer line part 332 that electrically connects the antenna unit Ant and the signal processing unit 10. The signal processing unit 10 may correct a phase difference between channels of signals transmitted and received in the transfer line part 332. In an embodiment, the electronic device 1 may be provided with a transmission transfer line part 332 that electrically connects the transmission antenna unit Ant and the transmission signal processing unit 10. The electronic device 1 may be provided with a reception transfer line part 332 that electrically connects the reception antenna unit Ant and the reception signal processing unit 10. The transmission signal processing unit 10 may also correct a phase difference between channels of signals transmitted in the transmission transfer line part 332. The reception signal processing unit 10 corrects a phase difference between channels of signals received in the reception transfer line part 332,

In an embodiment, the board on which the antenna unit Ant is mounted may be separate from the board 140 on which the signal processing unit 10 is mounted. In this case, the board on which the antenna unit Ant is mounted and the board 140 on which the signal processing unit 10 is mounted may be connected by the transfer line part 332. In an embodiment, a first board on which the transmission antenna unit Ant or the reception antenna unit Ant is mounted may be separate from a second board on which the transmission signal processing unit 10 or the reception signal processing unit 10 is mounted. The first board and the second board may also be connected by the transmission transfer line part 332 or the reception transfer line part 332.

In an embodiment, the signal processing unit 10 may detect a subject of monitoring on the basis of a transmission signal transmitted as a transmission wave from the antenna unit Ant and a received signal received by the antenna unit Ant as a reflected wave resulting from the transmission wave being reflected by the subject of monitoring. In an embodiment, the transmission signal processing unit 10 may also detect the subject of monitoring on the basis of a transmission signal transmitted as a transmission wave from the transmission antenna unit Ant. The reception signal processing unit 10 may also detect the subject of monitoring on the basis of a received signal received by the reception antenna unit Ant as a reflected wave resulting from the transmission wave being reflected by the subject of monitoring.

In an embodiment, the signal processing unit 10 may also correct a deviation in direction-of-arrival estimation due to a phase delay difference occurring between channels of signals transmitted and received in the transfer line part 332. In an embodiment, the transmission signal processing unit 10 or the reception signal processing unit 10 may also correct a deviation in direction-of-arrival estimation due to a phase delay difference occurring between transmission channels or reception channels.

In an embodiment, the signal processing unit 10 may also correct the amplitude of a signal detected by the signal processing unit 10 on the basis of loss in signals transmitted and received in the transfer line part 332, as described above. In an embodiment, the transmission signal processing unit 10 or the reception signal processing unit 10 may also correct the amplitude of a signal detected by the transmission signal processing unit 10 or the reception signal processing unit 1- on the basis of signal loss.

In an embodiment, the signal processing unit 10 may also correct a distance to a detected subject of monitoring on the basis of transmission and reception electrical lengths of signals in the transfer line part 332. In an embodiment, the transmission signal processing unit 10 or the reception signal processing unit 10 may also correct the distance to the subject of monitoring on the basis of the electrical length for a signal in the transmission transfer line part 332 or the reception transfer line part 332.

In an embodiment, the antenna unit Ant may also include a first antenna unit Ant-1 and a second antenna unit Ant-2. In this case, a first transfer line part 332 may connect the first antenna unit Ant-1 and the signal processing unit 10. A second transfer line part 332 may connect the second antenna unit Ant-2 and the signal processing unit 10. In this case, an RF switch 333 may also be provided to switch between the connection of the first antenna unit Ant-1 and the signal processing unit 10 and the connection of the second antenna unit Ant-2 and the signal processing unit 10. In an embodiment, the transmission antenna unit Ant may also include a first transmission antenna unit Ant-1 and a second transmission antenna unit Ant-2. In this case, a first transmission transfer line part 332 may connect the first transmission antenna unit Ant-1 and the transmission signal processing unit 10. A second transmission transfer line part 332 may connect the second transmission antenna unit Ant-2 and the transmission signal processing unit 10. In this case, the electronic device 1 may also be provided with a transmission RF switch 333 to switch between the connection of the first transmission antenna unit Ant-1 and the transmission signal processing unit 10 and the connection of the second transmission antenna unit Ant-2 and the transmission signal processing unit 10.

In an embodiment, the electronic device 1 may be provided with two antenna units Ant (Ant-1 and Ant-2), and may also be provided with three or more antenna units Ant (Ant-1, Ant-2, and Ant-n). In an embodiment, the electronic device 1 may be provided with two transmission antenna units Ant, and may also be provided with three or more transmission antenna units Ant.

In an embodiment, the electronic device 1 may also be provided with a low-noise amplifier LNA 331 that amplifies, while maintaining low noise, a received signal received by a receiving antenna in the antenna unit Ant. In an embodiment, the electronic device 1 may also be provided with a low-noise amplifier LNA 331 that amplifies, while maintaining low noise, a received signal received by a receiving antenna in the reception antenna unit Ant.

In an embodiment, the antenna unit Ant may also be a sheet antenna or the like formed on a flexible substrate. In an embodiment, the transmission antenna unit Ant and/or the reception antenna unit Ant may also be a sheet antenna formed on a flexible substrate.

As above, in an embodiment, the electronic device 1 performs frequency filtering on vibrations at the position where the test subject 200 is present to extract components thought to be the envelope curve of the heartbeat, and obtains the interval between peaks of the envelope curve as the heartbeat interval. In this way, in an embodiment, the electronic device 1 can calculate the heartbeat interval of the test subject 200.

For example, by being installed in a moving body such as an automobile, the electronic device 1 according to an embodiment can detect the heartbeat and the like of an occupant on board the moving body. In this case, the electronic device 1 according to an embodiment can analyze the RRI of a driver who is operating the moving body such as an automobile by performing radar-based heartbeat measurement on the driver. If such RRI analysis can be performed with relatively high precision, the electronic device 1 can be used to estimate the psychological state, including level of alertness, of an occupant such as the driver, for example. If such RRI analysis can be performed with relatively high precision, the electronic device 1 can also be used to detect distracted driving and the like, including the driver falling asleep at the wheel, or to detect predictive signs of such distracted driving.

As described above, according to an embodiment, the electronic device 1 can measure vibration on the body surface of a test subject by applying signal processing to a reflected wave (received wave) obtained from a radar. As a result, according to an embodiment, the electronic device 1 can well acquire a heart sound waveform of a human being or an animal. According to an embodiment, the electronic device 1 can use multivariate analysis to detect the presence or absence of noise, including road noise, superimposed on the acquired heart sound waveform. According to an embodiment, if noise is included in the acquired heart sound waveform, the electronic device 1 can separate the heart sound and the noise.

In an embodiment, the electronic device 1 can be expected to exhibit various technical effects as a result of the antenna unit Ant that includes an antenna for transmitting and receiving radio waves being implemented separately from the circuit unit 400 that includes an MMIC/SoC.

For example, as described above, in the case of detecting biological information such as the heartbeat of a user by transmitting and receiving radio waves, it may be necessary to control the directivity of the transmission wave and/or perform direction estimation processing on the received wave by using a sufficiently abundant number of transmitting and receiving antennas. In such a case, it is desirable to use an antenna with four or more channels for both transmission and reception. According to the electronic device 1 as in an embodiment, the overall size can be reduced by integrating the transmitting antenna array 24 and the receiving antenna array 31 together with the signal processing unit 10 that includes the MMIC/SoC.

As described above, in general, adequately and properly executing processing for a radar sensor as illustrated in FIG. 10 means that a CPU/DSP or other processor in a circuit unit (the signal processing unit 10) such as an SoC tends to generate more heat. Therefore, it is assumed that it may be necessary to increase the size of, for example, the heat dissipation mechanism in the electronic device 100 according to a comparative example of an embodiment.

However, the electronic device 1 according to an embodiment is compatible with situations where the configuration is to be mounted in a narrow space, such as in the case of sensing the vitals of a user inside the cabin of an automobile.

As described above, another concern is that, given a radar sensor configuration like the one illustrated in FIG. 10, for example, the area that is detectable by a single MMIC/SoC (the signal processing unit 10) may be limited. However, according to the electronic device 1 as in an embodiment, a plurality of antenna units Ant can be used to detect a relatively wide area, even in the case of using a single MMIC/SoC (signal processing unit 10).

As described above, according to the electronic device 1 as in an embodiment, a higher degree of freedom in the implementation of the electronic device 1 (or the antenna unit Ant and the circuit unit 400) can be achieved while also achieving good detection through the transmission and reception of radio waves, such as millimeter waves, for example. Consequently, according to an embodiment, an electronic device and the like that allow for enhanced convenience can be provided.

In an embodiment, the electronic device 1 can measure distance (perform distance measurement), measure an angle (perform angle measurement), and detect a Doppler velocity (perform velocity measurement) by using a Doppler radar operating at high frequencies (20 GHz or higher) such as the millimeter-wave band, for example. In an embodiment, the electronic device 1 can be used to detect a vital signal pertaining to the heart sound, heartbeat, respiration, or biological vibrations in a human being or an animal, or to detect vibrations in a building or a machine, for example. According to the electronic device 1 as in an embodiment, the transmitting and receiving antennas can be implemented separately, thereby enabling the implementation of a millimeter-wave radar or other system even in applications where little space is available.

(Other embodiments)

The following describes other embodiments.

The embodiment described above assumes that sensing is performed to detect the heartbeat/respiration/body movement and/or the like of a human being or an animal, for example. However, the sensing performed by the electronic device 1 according to an embodiment is not limited to the detection of the heartbeat/respiration/body movement and/or the like of a human being or an animal. For example, in an embodiment, the electronic device 1 can also be utilized in applications for measuring general vibrations in an artificial object or a natural object, for example.

As illustrated in FIGs. 11 and 12, in an embodiment, the electronic device 1 is described as being provided with a plurality of antenna units Ant, such as the antenna unit Ant-1, the antenna unit Ant-2, and so on. However, in an embodiment, the electronic device may also be provided with only one antenna unit, such as only Ant-1, for example.

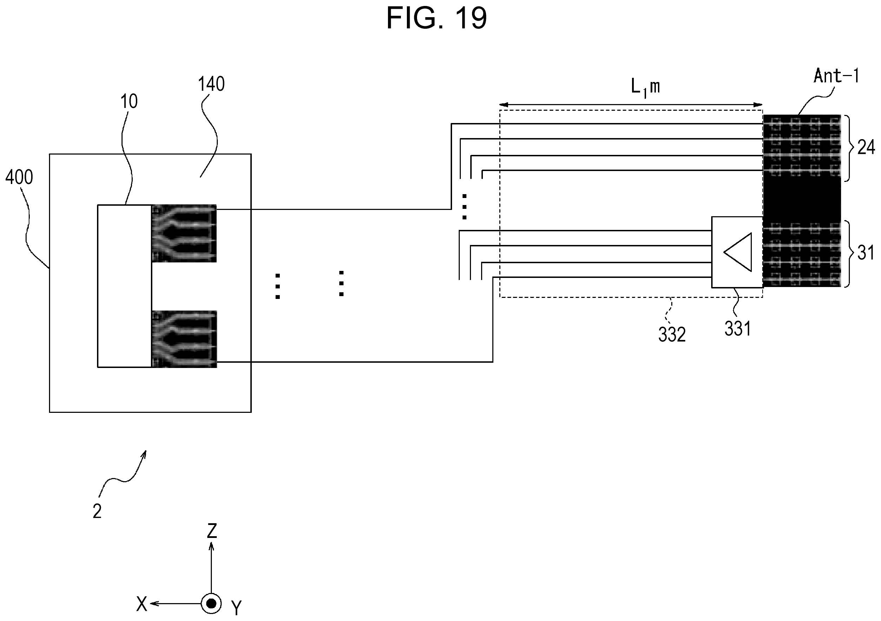

FIG. 19 is a diagram illustrating an example of the schematic structure of an electronic device provided with only one antenna unit.

As illustrated in FIG. 19, an electronic device 2 may be provided with only one antenna unit Ant, such as an antenna unit Ant-1. That is, the electronic device 2 illustrated in FIG. 19 may be provided with one antenna unit Ant-1 and one circuit unit 400. As illustrated in FIG. 19, the antenna unit Ant-1 and the circuit unit 400 may be electrically connected by the transfer line part 332. Since there is only one antenna unit Ant, the SPDT, SPnT, or other type of RF switch 333 can be omitted from the electronic device 2 illustrated in FIG. 19, as compared to the electronic device 1 illustrated in FIG. 11.

As illustrated in FIG. 12, in the electronic device 1 according to an embodiment, the antenna unit Ant-1 and/or the antenna unit Ant-2 may be encased in an enclosure, for example. On the other hand, in an electronic device according to an embodiment, at least one of one or more antenna units Ant may also include an exposed antenna that is not encased, for example. The exposed antenna may be an antenna formed using a rigid substrate, an antenna formed using a flexible substrate, a curved antenna that is not planar, or the like.

An electronic device such as the electronic device 1 according to an embodiment described above may execute amplitude correction, phase correction, or both. For example, if there are small amplitude differences between channels, an electronic device such as the electronic device 1 may execute only phase correction and omit amplitude correction.

In the embodiment described above, the orientation of the radiating surface of the antenna unit Ant and the orientation of any planar part of the circuit unit 400 may be set in various ways.

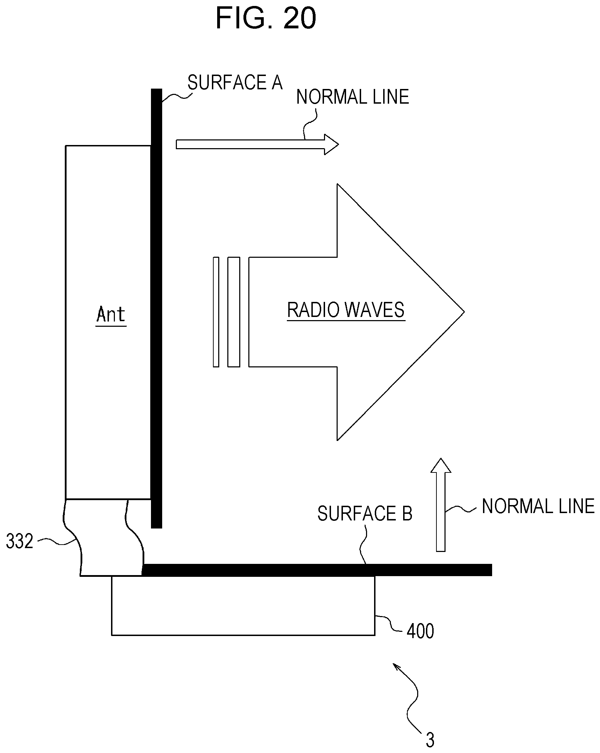

FIG. 20 is a diagram illustrating an example of the schematic structure of an electronic device according to a variability of an embodiment. An embodiment may also adopt a configuration like that of the electronic device 3 illustrated in FIG. 20, in which the normal direction of the radiating surface (surface A) of the antenna unit Ant and the normal direction of the front surface (surface B) of the circuit unit 400 including an SoC and/or the like are different (such that the normal lines are orthogonal, for example). Adopting such a configuration can be expected to produce an effect of allowing for a higher degree of freedom in installing the antenna unit Ant.

For reference, the following gives several example of installations of an electronic device such as the electronic device 1 according to the embodiment described above.

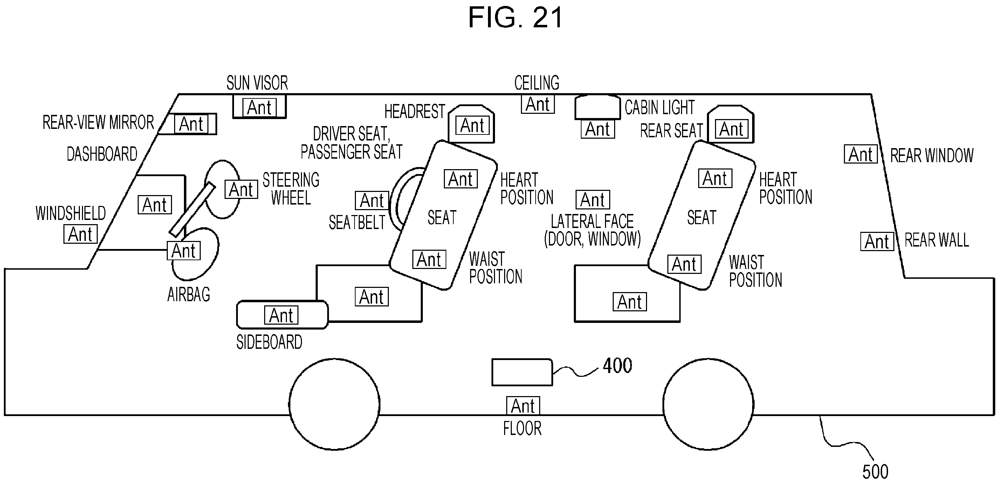

FIG. 21 is a diagram schematically illustrating an example of places where the antenna unit Ant of the electronic device 1 according to an embodiment can be installed in a moving body 500 such as an automobile. As illustrated in FIG. 21, the antenna unit Ant of the electronic device 1 according to an embodiment may be installed in at least one of the locations in the moving body 500 that correspond to the positions labeled "Ant" in the diagram. As illustrated in FIG. 21, in an embodiment, the circuit unit 400 of the electronic device 1 may be installed anywhere, such as in the vicinity of an ECU. Each antenna unit Ant and the circuit unit 400 may be electrically connected by the transfer line part 332. In FIG. 21, the transfer line part 332 connecting each antenna unit Ant and the circuit unit 400 is omitted from illustration.

As illustrated in FIG. 21, the antenna unit Ant may be installed at the position of (such as on the surface of, or inside) a headrest, a seat, or the like of the moving body 500, for example. According to such installation of the antenna unit Ant, installation space can be secured even in a confined space inside a vehicle. The antenna unit Ant installed in this way can be used to precisely detect vitals at a position near a person (occupant).

As illustrated in FIG. 21, antenna units Ant may also be installed at two positions on or in a seat of the moving body 500, such as at the positions of the waist and the heart of a seated human being. The antenna units Ant installed in this way can be used to detect occupants of various body sizes, such as both adults and children.

As illustrated in FIG. 21, the antenna unit Ant may also be installed at any of various positions at the front of the moving body 500, such as on or in a steering wheel, a dashboard, a sun visor, and/or a rear-view mirror of the moving body 500. The antenna unit Ant installed in this way can be used to accurately detect heartbeat in a chest area of an occupant.

As illustrated in FIG. 21, the antenna unit Ant may also be installed on or in the steering wheel and/or the dashboard of the moving body 500. According to such installation of the antenna unit Ant, installation space can be secured even in a confined space inside a vehicle. The antenna unit Ant installed in this way can be used to precisely detect vitals of the driver in particular.

As illustrated in FIG. 21, the antenna unit Ant may also be installed near a ceiling, a cabin light, and/or the like of the moving body 500. According to such installation of the antenna unit Ant, installation space can be secured even in a confined space inside a vehicle. The antenna unit Ant installed in this way can be used to perform detection over a wide range of the interior of the moving body 500.

As illustrated in FIG. 21, the antenna unit Ant may also be installed on or in a door, a floor, a rear window, and/or a rear part of the moving body 500. According to such installation of the antenna unit Ant, installation space can be secured even in a confined space inside a vehicle.

In the embodiment described above, the electronic device 1 may be installed in all types of moving bodies that move on public roads, for example. However, in another embodiment, an electronic device not only may be applied to moving bodies that move on public roads, such as automobiles, but may also be applied similarly in environments where noise is thought to be generated, such as aircraft, ships, or trains, for example. In another embodiment, an electronic device may also be applied similarly to noise generated by physical activity by a test subject in an indoor environment. In an embodiment, an electronic device may be applied to heartbeat measurement and/or the like for a test subject riding a motorcycle, a bus, a bicycle, a truck, a construction vehicle, or a tractor, for example.

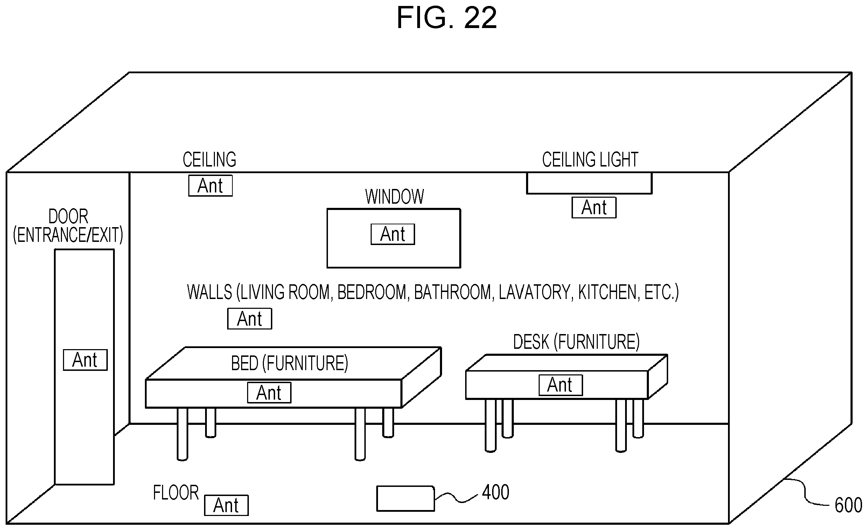

FIG. 22 is a diagram schematically illustrating an example of places where the antenna unit Ant of the electronic device 1 according to an embodiment can be installed in a space 600 such as a residence or an office. As illustrated in FIG. 22, the antenna unit Ant of the electronic device 1 according to an embodiment may be installed in at least one of the locations in the space 600 that correspond to the positions labeled "Ant" in the diagram. As illustrated in FIG. 22, in an embodiment, the circuit unit 400 of the electronic device 1 may be installed anywhere, such as under the floor. Each antenna unit Ant and the circuit unit 400 may be electrically connected by the transfer line part 332. In FIG. 22, the transfer line part 332 connecting each antenna unit Ant and the circuit unit 400 is omitted from illustration.

As illustrated in FIG. 22, the antenna unit Ant may be installed on or in a door, a floor, a wall, and/or the like in the space 600, for example. The antenna unit Ant installed in this way can be used to detect a human being, an animal, or the like entering or exiting the space 600, such as a person.

As illustrated in FIG. 22, the antenna unit Ant may be installed inside or near a ceiling, a ceiling light, and/or the like in the space 600, for example. The antenna unit Ant installed in this way can be used to monitor the whole interior of the space 600 from above.

As illustrated in FIG. 22, the antenna unit Ant may be installed inside or near a piece of furniture or the like in the space 600, for example. The antenna unit Ant installed in this way can also be moved within the space 600.

As illustrated in FIG. 22, the antenna unit Ant may be installed inside or near a bed in the space 600, for example. As illustrated in FIG. 22, the antenna unit Ant may be installed near the ceiling or the like opposite the bed in the space 600, for example. As illustrated in FIG. 22, the antenna unit Ant may be installed on or near a frame of the bed in the space 600, for example. The antenna unit Ant installed in this way can be used to continuously measure the heartbeat, respiration, and/or the like of a human being resting on the bed in the space 600.

As illustrated in FIG. 22, antenna units Ant may also be installed on the four sides of lateral wall surfaces in the space 600, for example. In this case, the antenna units Ant may be installed on the lateral wall surfaces, inside the lateral wall surfaces, and/or the like. The space 600 may be a living room, a bedroom, a bathroom, a lavatory, or a kitchen, for example. The antenna unit Ant installed in this way can be used to continuously measure the heartbeat, respiration, and/or the like of a human being occupying the space 600. For example, this allows for continuous measurement of the heartbeat, respiration, and/or the like of a human being occupying the space 600, irrespective of whether the human being is watching television, resting, taking a bath, using the lavatory, cooking, or the like.

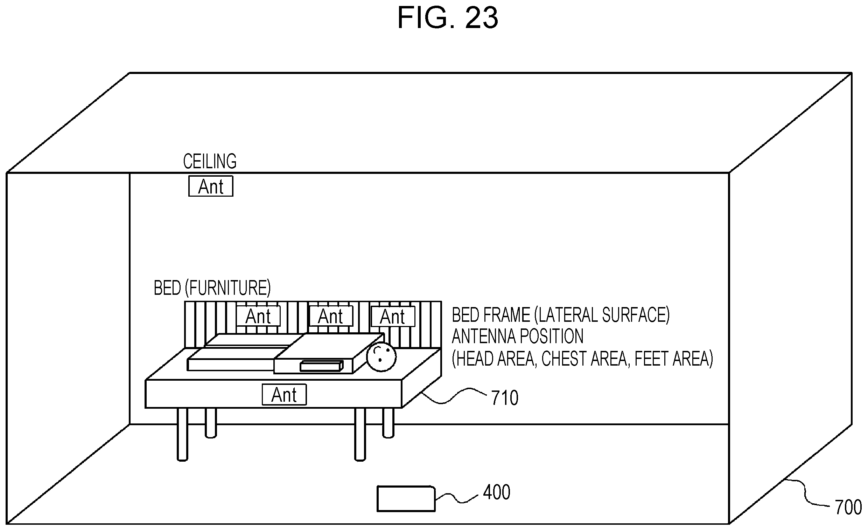

FIG. 23 is a diagram schematically illustrating an example of places where the antenna unit Ant of the electronic device 1 according to an embodiment can be installed in a space 700 such as a hospital room or a nursing home. As illustrated in FIG. 23, the antenna unit Ant of the electronic device 1 according to an embodiment may be installed in at least one of the locations in the space 700 that correspond to the positions labeled "Ant" in the diagram. As illustrated in FIG. 23, in an embodiment, the circuit unit 400 of the electronic device 1 may be installed anywhere, such as under the floor. Each antenna unit Ant and the circuit unit 400 may be electrically connected by the transfer line part 332. In FIG. 23, the transfer line part 332 connecting each antenna unit Ant and the circuit unit 400 is omitted from illustration.

As illustrated in FIG. 23, the antenna unit Ant may be installed inside or near a bed 710 in the space 700, for example. As illustrated in FIG. 23, the antenna unit Ant may be installed near the ceiling or the like opposite the bed 710 in the space 700, for example. As illustrated in FIG. 23, the antenna unit Ant may be installed inside or near a frame (or lateral surface) of the bed 710 in the space 700, for example. In this case, as illustrated in FIG. 23, the antenna unit Ant may be installed near a position that corresponds to a head area, a chest area, a leg area (or foot area), and/or the like of a human being lying on the bed 710, for example. The antenna unit Ant installed in this way can be used to vitals more precisely according to the position of the body of a human being or the like lying on the bed 710.

In the electronic device 1 illustrated in FIG. 2, the signal processing unit 10 is described as being provided with functional units such as the heartbeat extraction unit 13 and the calculation unit 14. However, in an embodiment, the processing performed by the heartbeat extraction unit 13 and/or the calculation unit 14 may also be performed by an external computer, processor, or the like.

As described above, in an embodiment, the electronic device 1 uses, for example, a millimeter-wave sensor provided with a plurality of transmitting antennas and a plurality of receiving antennas to detect weak vibrations such as a heartbeat. In an embodiment, when the subject is not detected, the electronic device 1 detects the body motion of the subject while varying the transmission phase of the antennas to create a beamforming pattern of the transmitting antennas. On the other hand, in an embodiment, once the body motion of the subject is detected, the electronic device 1 carries out beamforming in the direction of the body motion to detect the heartbeat. In this way, according to an embodiment, the electronic device 1 can improve the signal quality by automatically detecting the direction of a human body. Consequently, according to an embodiment, the electronic device 1 can improve the heartbeat detection precision and/or detection range. Thus, according to an embodiment, the electronic device 1 can detect the heartbeat of a human being with high precision.

The present disclosure has been described on the basis of the drawings and examples, but note that a person skilled in the art could easily make various variations or revisions on the basis of the present disclosure. Consequently, it should be understood that these variations or revisions are included in the scope of the present disclosure. For example, the functions and the like included in each functional unit may be rearranged in logically non-contradictory ways. Multiple functional units or the like may be combined into one, or a functional unit may be divided. Each embodiment according to the present disclosure described above is not limited to being carried out exactly according to each embodiment as described, and may be carried out by combining features or omitting some features, as appropriate. In other words, the content of the present disclosure enables a person skilled in the art to make various variations and revisions on the basis of the present disclosure. Therefore, these variations and revisions are included in the scope of the present disclosure. For example, in each embodiment, each functional unit, means, step, and the like can be added to another embodiment or replaced by each functional unit, means, step, and the like of another embodiment in logically non-contradictory ways. In each embodiment, multiple functional units, means, steps, and the like can be combined into one, or each functional unit, means, step, and the like can be divided. Each embodiment according to the present disclosure described above is not limited to being carried out exactly according to each embodiment as described, and can be carried out by combining features or omitting some features, as appropriate.

The embodiments described above are not limited solely to embodiments of the electronic device 1. For example, the embodiments described above may also be carried out as a method of controlling a device like the electronic device 1. Furthermore, the embodiments described above may also be carried out as a program to be executed by a device like the electronic device 1, for example, or as a storage medium or recording medium in which the program is recorded.

REFERENCE SIGNS

| | |
|---|---|
| 1 | electronic device |
| 10 | signal processing unit |
| 11 | signal generation processing unit |
| 12 | received signal processing unit |
| 13 | heartbeat extraction unit |
| 14 | calculation unit |
| 21 | transmission DAC |
| 22 | transmission circuit |
| 23 | millimeter-wave transmission circuit |
| 24 | transmitting antenna array |
| 31 | receiving antenna array |
| 32 | mixer |
| 33 | reception circuit |
| 34 | reception ADC |
| 40A, 40B | waveguide |
| 50 | communication interface |
| 60 | external device |
| 100 | electronic device (comparative example) |
| 110 | enclosure |
| 120 | radome unit |
| 130 | board |
| Ant | antenna unit |
| 140 | board |
| 331 | LNA |
| 332 | transfer line part |
| 333 | RF switch |
| 400 | circuit unit |
| 500 | moving body |
| 600 | space |
| 700 | space |
| 710 | bed |

**Claims**

**1.**    An electronic device comprising:

a transmission antenna unit configured to have a plurality of transmission channels;

a transmission signal processing unit configured to generate a transmission signal to be transmitted from the transmission antenna unit; and

a transmission transfer line part configured to electrically connect the transmission antenna unit and the transmission signal processing unit, wherein

the transmission signal processing unit is configured to correct a phase difference between channels of signals transmitted in the transmission transfer line part.

2. The electronic device according to claim 1, comprising:

a reception antenna unit configured to have a plurality of reception channels;

a reception signal processing unit configured to process a received signal received from the reception antenna unit; and

a reception transfer line part configured to electrically connect the reception antenna unit and the reception signal processing unit, wherein

the reception signal processing unit is configured to correct a phase difference between channels of signals received in the reception transfer line part.

3. The electronic device according to claim 2, wherein

a first board on which the transmission antenna unit or the reception antenna unit is mounted is separate from a second board on which the transmission signal processing unit or the reception signal processing unit is mounted, and

the first board and the second board are connected by the transmission transfer line part or the reception transfer line part.

4. The electronic device according to claim 1, wherein
the transmission signal processing unit is configured to detect a subject of monitoring on the basis of a transmission signal transmitted as a transmission wave from the transmission antenna unit.

5. The electronic device according to claim 4, wherein
the reception signal processing unit is configured to detect the subject of monitoring on the basis of a received signal received by the reception antenna unit as a reflected wave resulting from the transmission wave being reflected by the subject of monitoring.

6. The electronic device according to claim 2, wherein
the transmission signal processing unit or the reception signal processing unit is configured to correct a deviation in direction-of-arrival estimation due to a phase delay difference occurring between transmission channels or reception channels.

7. The electronic device according to claim 2, wherein
the transmission signal processing unit or the reception signal processing unit is configured to correct the amplitude of a signal detected by the transmission signal processing unit or the reception signal processing unit on the basis of signal loss.

8. The electronic device according to claim 2, wherein
the transmission signal processing unit or the reception signal processing unit is configured to correct the distance to the subject of monitoring on the basis of the electrical length for a signal in the transmission transfer line part or the reception transfer line part.

9. The electronic device according to claim 1, wherein

the transmission antenna unit is configured to include a first transmission antenna unit and a second transmission antenna unit, and

the electronic device comprises:

a first transmission transfer line part configured to connect the first transmission antenna unit and the transmission signal processing unit;

a second transmission transfer line part configured to connect the second transmission antenna unit and the transmission signal processing unit; and

a transmission switch configured to switch between the connection of the first transmission antenna unit and the transmission signal processing unit and the connection of the second transmission antenna unit and the transmission signal processing unit.

10. The electronic device according to claim 1, comprising:
two transmission antenna units.

11. The electronic device according to claim 1, comprising:
three or more transmission antenna units.

12. The electronic device according to claim 2, comprising:
a low-noise amplifier configured to amplify, while maintaining low noise, a received signal received by a receiving antenna in the reception antenna unit.

13. The electronic device according to claim 2, wherein
the transmission antenna unit and/or the reception antenna unit is a sheet antenna formed on a flexible substrate.

14. A method of controlling an electronic device, the electronic device being provided with

a transmission antenna unit configured to have a plurality of transmission channels,
a transmission signal processing unit configured to generate a transmission signal to be transmitted from the transmission antenna unit, and
a transmission transfer line part configured to electrically connect the transmission antenna unit and the transmission signal processing unit,
the method comprising:
correcting, by the transmission signal processing unit, a phase difference between channels of signals transmitted in the transmission transfer line part.

15. A program causing an electronic device to execute a process, the electronic device being provided with

a transmission antenna unit configured to have a plurality of transmission channels,
a transmission signal processing unit configured to generate a transmission signal to be transmitted from the transmission antenna unit, and
a transmission transfer line part configured to electrically connect the transmission antenna unit and the transmission signal processing unit,
the process comprising:
correcting, by the transmission signal processing unit, a phase difference between channels of signals transmitted in the transmission transfer line part.

# FIG. 1

# FIG. 2

EP 4 772 903 A1

# FIG. 3

# FIG. 4

SUBFRAME 1

SUBFRAME N

# FIG. 5

# FIG. 6

# FIG. 7

# FIG. 8

$d_{1,t} < \dfrac{\lambda}{2}$

24

$d_{2,t} < \dfrac{\lambda}{2}$

$d_{1,s} < \dfrac{\lambda}{2}$

31

$d_{2,s} < \dfrac{\lambda}{2}$

DIRECTION 2

DIRECTION 1

# FIG. 9

# FIG. 10

FIG. 11

Ant-1  24  31
Ant-2  24  31

331
332
L₁m
L₂m
333
333
140
40A
10
40B
400
1

X
Z
Y

FIG. 12

# FIG. 13

# FIG. 14

| | |
|---|---|
| -------- | S11 CONDUCTION LINE PART 60 cm |
| -·--·-- | S11 CONDUCTION LINE PART 61 cm |

## FIG. 15A

AMPLITUDE

## FIG. 15B

PHASE

## FIG. 16A

AMPLITUDE

## FIG. 16B

PHASE

# FIG. 17A

AMPLITUDE

Frequency[GHz]

dB(S$_{11}$) CONDUCTION LINE PART 60 cm
dB(S$_{11}$) CONDUCTION LINE PART 61 cm

# FIG. 17B

PHASE

Frequency[GHz]

angle(S$_{11}$) CONDUCTION LINE PART 60 cm
angle(S$_{11}$) CONDUCTION LINE PART 61 cm

# FIG. 18

START

CORRECT DATA IN EACH CHANNEL — S100

PROCESS RECEIVED SIGNAL — S110

EXTRACT SIGNAL SOURCE — S120

CONVERT TO VIBRATION WAVEFORM — S130

EXTRACT VIBRATION DATA — S140

CALCULATE RRI — S150

CALCULATE HRV — S160

END

# FIG. 19

EP 4 772 903 A1

# FIG. 20

SURFACE A

NORMAL LINE

Ant

RADIO WAVES

332

SURFACE B

NORMAL LINE

400

3

FIG. 21

# FIG. 22

CEILING

Ant

CEILING LIGHT

Ant

WINDOW

Ant

DOOR
(ENTRANCE/EXIT)

Ant

WALLS (LIVING ROOM, BEDROOM, BATHROOM, LAVATORY, KITCHEN, ETC.)

Ant

DESK (FURNITURE)

BED (FURNITURE)

Ant

Ant

FLOOR Ant

400

600

EP 4 772 903 A1

# FIG. 23

CEILING
Ant

BED (FURNITURE)

Ant    Ant    Ant

BED FRAME (LATERAL SURFACE)
ANTENNA POSITION
(HEAD AREA, CHEST AREA, FEET AREA)

Ant

710

400

700

EP 4 772 903 A1

## INTERNATIONAL SEARCH REPORT

International application No.

**PCT/JP2024/028133**

### A. CLASSIFICATION OF SUBJECT MATTER

*G01S 7/40*(2006.01)i; *A61B 5/11*(2006.01)i; *G01S 7/02*(2006.01)i; *G01S 13/34*(2006.01)i
FI:    G01S7/40 117; A61B5/11 110; G01S13/34; G01S7/02 216

According to International Patent Classification (IPC) or to both national classification and IPC

### B. FIELDS SEARCHED

Minimum documentation searched (classification system followed by classification symbols)

G01S 7/00 - G01S 7/42; G01S 13/00 - G01S 13/95

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2020-024185 A (ASAHI KASEI EMD CORPORATION) 13 February 2020 (2020-02-13) paragraphs [0123], [0131], [0155]-[0159], [0229]-[0277], fig. 19A-19C, 27-30 | 1-2, 4-6, 12, 14-15 |
| Y | | 3, 7-11, 13 |
| X | WO 2018/181201 A1 (NEC CORPORATION) 04 October 2018 (2018-10-04) paragraphs [0047], [0113]-[0119], fig. 17 | 1-2, 4-5, 12, 14-15 |
| Y | WO 2021/106418 A1 (HITACHI ASTEMO, LTD.) 03 June 2021 (2021-06-03) paragraphs [0016]-[0017], [0028], [0041]-[0042], fig. 4, 8 | 3, 13 |
| Y | US 2005/0181784 A1 (HO, Chiacheng) 18 August 2005 (2005-08-18) paragraphs [0020]-[0027], [0033], fig. 2-3 | 7 |
| Y | JP 2007-033093 A (JAPAN RADIO CO., LTD.) 08 February 2007 (2007-02-08) paragraph [0015] | 8 |

☑ Further documents are listed in the continuation of Box C.     ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **21 August 2024** | **10 September 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**EP 4 772 903 A1**

## INTERNATIONAL SEARCH REPORT

International application No.

**PCT/JP2024/028133**

### C. DOCUMENTS CONSIDERED TO BE RELEVANT

| Category* | Citation of document, with indication, where appropriate, of the relevant passages | Relevant to claim No. |
|---|---|---|
| Y | JP 2023-102666 A (PANASONIC INTELLECTUAL PROPERTY MANAGEMENT CO., LTD.) 25 July 2023 (2023-07-25)<br>paragraphs [0035]-[0064], fig. 3 | 9 |
| Y | JP 2016-121986 A (PANASONIC INTELLECTUAL PROPERTY MANAGEMENT CO., LTD.) 07 July 2016 (2016-07-07)<br>paragraphs [0013]-[0025], [0037], fig. 3, 5 | 10-11 |
| A | CN 111596266 A (XIDIAN UNIVERSITY) 28 August 2020 (2020-08-28)<br>paragraphs [0046]-[0085], fig. 1-5 | 1-15 |
| A | CN 104360327 A (BEIJING INSTITUTE OF TECHNOLOGY) 18 February 2015 (2015-02-18)<br>paragraphs [0100]-[0150], fig. 2-4 | 1-15 |

Form PCT/ISA/210 (second sheet) (July 2022)

## INTERNATIONAL SEARCH REPORT
### Information on patent family members

International application No.

**PCT/JP2024/028133**

| Patent document cited in search report | | | Publication date (day/month/year) | Patent family member(s) | | | Publication date (day/month/year) |
|---|---|---|---|---|---|---|---|
| JP | 2020-024185 | A | 13 February 2020 | US 2019/0391249 A1 paragraphs [0196], [0204], [0228]-[0232], [0309]-[0357], fig. 19A-19C, 27-30<br>JP 7319827 B2 | | | |
| WO | 2018/181201 | A1 | 04 October 2018 | (Family: none) | | | |
| WO | 2021/106418 | A1 | 03 June 2021 | JP 2021-085775 A<br>JP 7370829 B2<br>DE 112020004943 T5 | | | |
| US | 2005/0181784 | A1 | 18 August 2005 | TW 200529573 A | | | |
| JP | 2007-033093 | A | 08 February 2007 | (Family: none) | | | |
| JP | 2023-102666 | A | 25 July 2023 | (Family: none) | | | |
| JP | 2016-121986 | A | 07 July 2016 | US 2016/0187479 A1 paragraphs [0019]-[0031], [0043], fig. 3, 5<br>CN 105738871 A | | | |
| CN | 111596266 | A | 28 August 2020 | (Family: none) | | | |
| CN | 104360327 | A | 18 February 2015 | (Family: none) | | | |

Form PCT/ISA/210 (patent family annex) (July 2022)

**EP 4 772 903 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023140490 A

- JP 2022020270 A